(19) Europäisches Patentamt
European Patent Office
Office européen des brevets

(11) **EP 4 342 463 A1**

(12) **EUROPEAN PATENT APPLICATION**

(43) Date of publication:
**27.03.2024 Bulletin 2024/13**

(21) Application number: **23198589.6**

(22) Date of filing: **20.09.2023**

(51) International Patent Classification (IPC):
***A61K 31/192*** *(2006.01)* ***A61K 9/00*** *(2006.01)*
***A61P 27/02*** *(2006.01)* ***A61P 35/00*** *(2006.01)*
***A61P 35/04*** *(2006.01)* ***A61K 38/17*** *(2006.01)*

(52) Cooperative Patent Classification (CPC):
(C-Sets available)
**A61K 31/192; A61K 9/0019; A61K 38/1774; A61P 27/02; A61P 35/00; A61P 35/04** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.09.2022 CN 202211155404**

(71) Applicant: **Guangzhou AnHao Pharmaceutical Technology Co., Ltd.**
**Huangpu Guangdong 510535 (CN)**

(72) Inventor: **YONG, Zhiquan**
**Huangpu District, Guangzhou (CN)**

(74) Representative: **Altmann Stößel Dick Patentanwälte PartG mbB**
**Theodor-Heuss-Anlage 2**
**68165 Mannheim (DE)**

(54) **STILBENE COMPOUNDS FOR USE IN THE TREATMENT OF TUMOUR AND EYE DISEASES**

(57) Provided is the use of stilbene compounds in the preparation of anti-tumor medicaments, and related is the pharmaceutical field. The stilbene compounds of the present invention are compounds represented by formula I, or salts thereof, or stereoisomers thereof, or solvates thereof. In the present invention, it is discovered that stilbene compounds have a dual effect of enriching T lymphocytes $CD8^+$ in solid tumors and targeting the destruction of the inner wall of tumor blood vessels. While killing tumor cells by cytotoxic T lymphocyte antigen $CD8^+$, the stilbenes cut off the blood supply to tumor tissues, cause rapid necrosis in solid tumors, greatly improve the killing effects on tumors, and inhibit tumor metastasis. Thereby, they can be used to prepare anti-tumor medicaments, and also used in combination with tumor immunotherapeutics to achieve synergistic anti-tumor effects. In addition, the stilbene compounds of the present invention can inhibit the growth of ocular surface blood vessels and be used in the preparation of medicaments for treating various eye diseases. The stilbene compounds of the present invention have good application prospects.

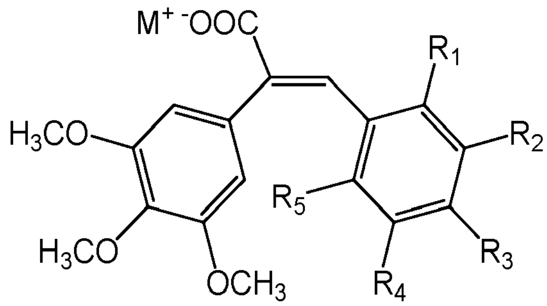

Formula I

EP 4 342 463 A1

(52) Cooperative Patent Classification (CPC): (Cont.)

C-Sets
**A61K 31/192, A61K 2300/00**

## Description

### Technical field

The present invention relates to the field of pharmaceuticals, and in particular to the use of stilbene compounds in the preparation of anti-tumor medicaments.

### Background technology

$CD8^+$ T lymphocytes are cytotoxic T lymphocytes, and can kill tumor cells and block the spread of tumors. Following administration of a medicament, if the concentration of $CD8^+$ T lymphocytes in solid tumor tissues is significantly increased, that is, the solid tumor tissue is rich in $CD8^+$ T lymphocytes, the anticancer effect of the medicament will be achieved.

In addition, the internal blood supply of malignant tumors and their surrounding blood vessels are abundant. If a medicament can act on vascular endothelial cells of tumors, and cause active blood vessels to undergo inflammatory reactions, the vessels will swell and be blocked, and thus cutting off the blood flow and the nutrition supply in the tumors can make tumor cells gradually die of starving, thereby, the antitumor effects can be reached.

In the previous investigation, the present inventors have found that styrene acid derivatives have a certain specific targeting effect on tumor blood vessels, can inhibit tumor growth, but cannot kill tumor cells, especially those at the edge of solid tumor tissues, and thus the tumor tissues crossing the edge may rapidly proliferate, resulting in poor therapeutic effects on tumors.

If a medicament is provided that can enrich $CD8^+$ T lymphocytes in solid tumor tissues and block the blood flow in tumors, it will have great application prospects for anti-tumor.

### Summary of the invention

The object of the present invention is to provide the use of stilbene compounds in the preparation of anti-tumor medicaments.

The present invention provides the use of a compound represented by formula I, or salts thereof, or stereoisomers thereof, or solvates thereof in the preparation of anti-tumor medicaments and/or medicaments for preventing and/or treating eye diseases:

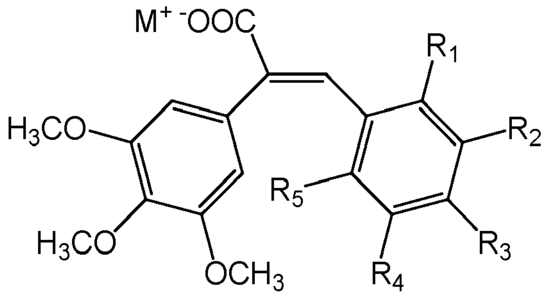

Formula I

wherein, M is selected from the group consisting of hydrogen, sodium, potassium, calcium, ammonium or an organic amine;

$R_1$, $R_2$, $R_3$, $R_4$, and $R_5$ are each independently selected from the group consisting of hydrogen, $C_1$-$C_6$ alkyl, $C_1$-$C_6$ alkoxy, hydroxy, amino, and halogen.

Further, the organic amine is selected from the group consisting of amino acids, peptides, tert-butylamine or n-butylamine;

and/or, $R_1$, $R_2$, $R_3$, $R_4$, and $R_5$ are each independently selected from the group consisting of hydrogen, $C_1$-$C_3$ alkyl, methoxy, ethoxy, hydroxy, amino, fluorine, chlorine or bromine;

preferably,

the organic amine is selected from the group consisting of meglumine, pyrazine, alanine, n-butylamine, lysine and tert-butylamine;

and/or, $R_1$, $R_2$, $R_3$, $R_4$, and $R_5$ are each independently selected from the group consisting of hydrogen, methoxy, ethoxy, hydroxy, fluorine, chlorine or bromine.

Further, the compound is represented by formula II:

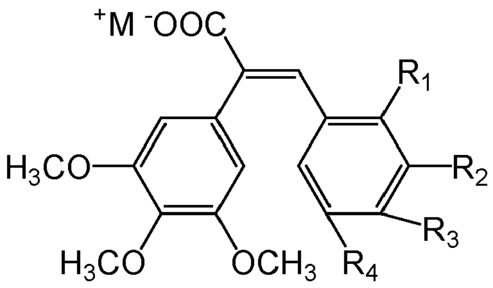

Formula II

wherein, M is selected from the group consisting of hydrogen, sodium, potassium, calcium, ammonium, meglumine, pyrazine, alanine, n-butylamine, lysine or tert-butylamine;
$R_1$, $R_2$, $R_3$, and $R_4$ are each independently selected from the group consisting of hydrogen, methoxy, ethoxy, hydroxy, fluorine, chlorine or bromine.

Further, the compound is represented by formula III:

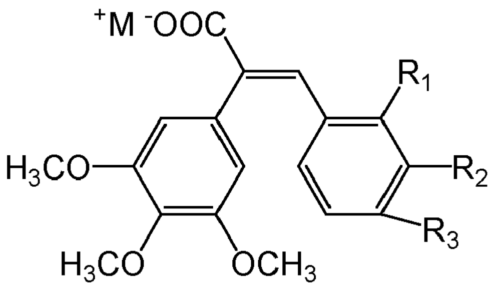

Formula III

wherein, M is selected from the group consisting of hydrogen, sodium, potassium, calcium, ammonium, meglumine, pyrazine, alanine, n-butylamine, lysine or tert-butylamine;
$R_1$, $R_2$, and $R_3$ are each independently selected from the group consisting of hydrogen, methoxy, ethoxy, hydroxy, fluorine, chlorine or bromine.

Further, the compound is represented by formula IV:

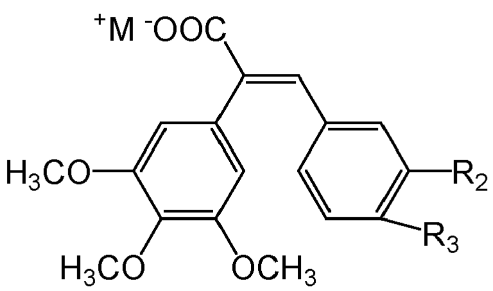

Formula IV

wherein, M is selected from the group consisting of hydrogen, sodium, potassium, calcium, ammonium, meglumine, pyrazine, alanine, n-butylamine, lysine or tert-butylamine;
$R_2$ and $R_3$ are each independently selected from the group consisting of hydrogen, methoxy, ethoxy, hydroxy, fluorine, chlorine or bromine.

Further, the anti-tumor medicament is a medicament that has the effect of enriching $CD8^+$ T lymphocytes in solid tumors and/or destroying the inner wall of tumor blood vessels;
preferably, the medicament is a medicament that can cause the necrosis of tumor cells at the centers of solid tumors.
Further, the anti-tumor medicament is a medicament that can inhibit tumor metastasis.
Further, when the anti-tumor medicament is a medicament that can inhibit tumor metastasis, the structure of the compound represented by formula I is as shown by formula Va:

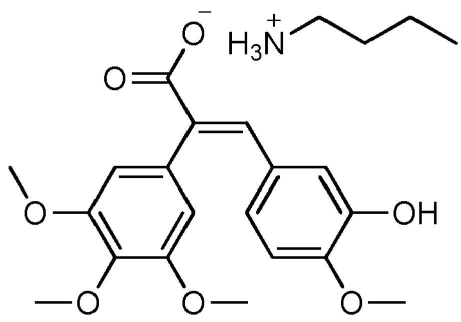

Formula Va;

preferably, the medicament inhibiting tumor metastasis is a medicament inhibiting melanoma metastasis.
Further, the anti-tumor medicament is a medicament for preventing and/or treating lung cancer, liver cancer, gastric cancer, ovarian cancer, prostate cancer, or renal cell carcinoma.
Further, when the anti-tumor medicament is a medicament for preventing and/or treating lung cancer, liver cancer, gastric cancer, or ovarian cancer, the structure of the compound represented by formula I is as shown by formula Va:

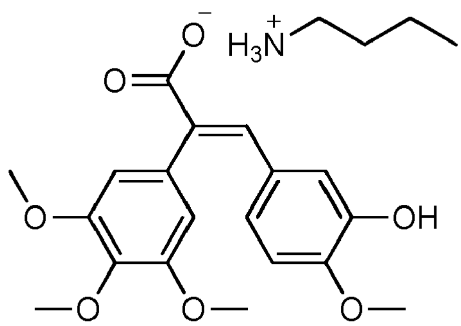

Formula Va.

Further, when the anti-tumor medicament is a medicament for preventing and/or treating prostate cancer or renal cell carcinoma, the structure of the compound represented by formula I is as shown by formula Vb:

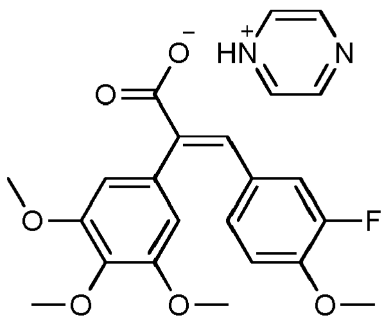

Formula Vb.

Further, the medicament for preventing and/or treating eye diseases is a medicament that inhibits the growth of ocular surface blood vessels.

Further, the eye diseases are retinopathy, fundus hemangioma, fundus hemorrhage, dacryocystitis, glaucoma, cataract, vitreous opacity, optic atrophy, macular degeneration and/or retinal detachment.

Further, the eye diseases are diabetic ophthalmopathy;
preferably, the diabetic ophthalmopathy is retinopathy, fundus hemangioma, fundus hemorrhage, dacryocystitis, glaucoma, cataract, vitreous opacity, optic atrophy, macular degeneration and/or retinal detachment related to diabetes.

Further, when the medicament is a medicament for preventing and/or treating eye diseases, the structure of the compound is represented by formula Va:

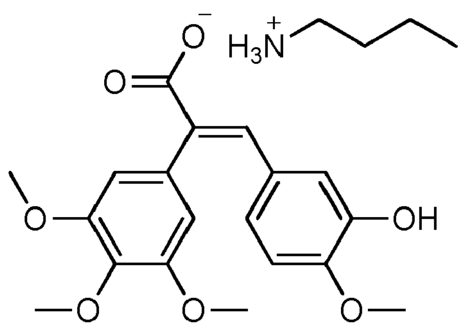

Formula Va.

The present invention also provides a pharmaceutical preparation for treating tumors, which is a pharmaceutical preparation prepared with the above-mentioned compound, or a salt thereof, or a stereoisomer thereof, or a solvate thereof, as the active ingredient, in combination with the pharmaceutically acceptable excipients or auxiliary ingredients; preferably, the pharmaceutical preparation is an injecting preparation, an oral preparation, or an external preparation.

The present invention also provides a pharmaceutical preparation for preventing and/or treating eye diseases, which is a pharmaceutical preparation prepared with the above-mentioned compound, or a salt thereof, or a stereoisomer thereof, or a solvate thereof, as the active ingredient, in combination with the pharmaceutically acceptable excipients or auxiliary ingredients;

preferably, the pharmaceutical preparation is a preparation administered by ophthalmic delivery;
more preferably, the dosage form of the preparation is eye drops, eye ointment, eye gel, nano preparation, microsphere preparation, and liposome preparation;
further preferably, the eye drops are aqueous solutions, suspensions, and emulsions

The present invention also provides the use of above-mentioned compound, or a salt thereof, or a stereoisomer thereof, or a solvate thereof, in combination with tumor immunotherapeutics, in the preparation of anti-tumor pharmaceutical compositions;
preferably, the tumor immunotherapeutics are PD1, PD-L1, T cells, NK cells, or CART cells.

Further, the compound represented by formula Vc, or a salt thereof, or a stereoisomer thereof, or a solvate thereof is used in combination with PD-L1 in the preparation of pharmaceutical compositions for preventing and/or treating lung cancers;

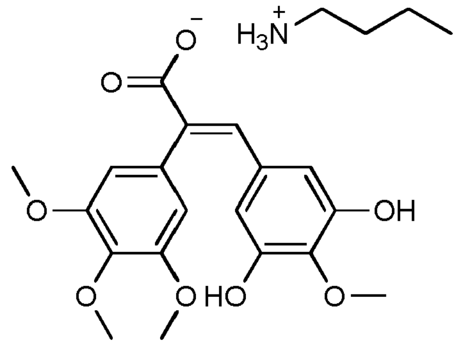

Formula Vc.

Further, the compound represented by formula Va, or a salt thereof, or a stereoisomer thereof, or a solvate thereof is used in combination with T cells in the preparation of pharmaceutical compositions for preventing and/or treating liver cancers;

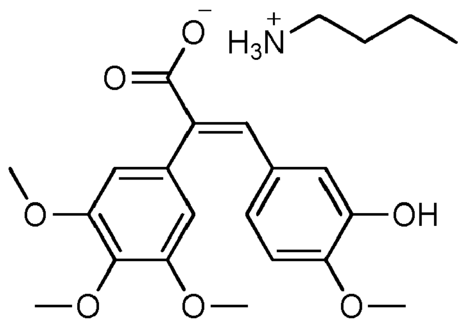

Formula Va.

In the previous experiments, the inventors of the present invention have found that styrene acid derivatives have a certain specific targeting effect on tumor blood vessels, can only destroy tumor blood vessels, inhibit tumor growth, but cannot kill tumor cells, especially those at the edge of solid tumor tissues, and thus the tumor tissues crossing the edge may rapidly proliferate. On the basis of previous work, the inventors have developed a series of compounds of the present invention by structural optimization and *in vivo* screening, which have dual effects of enriching $CD8^+$ T lymphocytes in solid tumor and destroying adhesion proteins of neovascular endothelial cells, and can be used to prepare anti-tumor medicaments.

The stilbene compounds with the structure of formula (I) can be used as the effective active ingredient, in combination with pharmaceutically acceptable excipients or carriers and other auxiliary materials, and processed according to corresponding general pharmaceutical procedures, and prepared as corresponding forms of vascular targeting medicaments such as oral, injection, or topical formulations. For example, after mixing with commonly used auxiliary materials such as disintegrating agents, excipients, lubricants, adhesives, and fillers that is acceptable for oral formulations, the mixture containing the compound of the present invention is processed according to the corresponding general procedures, and then oral medicaments can be obtained in the form of tablets, pills, granules, capsules, or appropriate forms of sustained release formulations, controlled release formulations, and other solid formulations; after mixing with suitable solvents and excipients that are acceptable for injection formulations and being processed as the corresponding procedures, the compound of the present invention can be prepared as intramuscular or intravenous injection formulations such as injection or powder injection; the compound of the present invention can be mixed with common solvents, stabilizers or sodium polyacrylate, polyvinyl alcohol, polyethylene glycol, glycerin, vegetable oil, vaseline, lanolin and other lipophilic and/or water-soluble bases, and processed according to corresponding procedures of topical medicaments, then prepared as eye drops or adhesive plasters, patches, gels, ointment, liniment, plastics, etc.. These injection formulations (e.g. injections, sterile powder injections, and sterile freeze-dried powder injections), oral formulations (e.g. tablets, capsules, various sustained and controlled release formulations), as well as topical formulations (e.g. eye drops and ointments), can be separately used as a medicament for the treatment of solid tumors.

The present invention provides the use of stilbene compounds in the preparation of anti-tumor medicaments. In the present invention, it is found that stilbene compounds have a dual effect of enriching $CD8^+$ T lymphocytes in solid tumors and targeting to damage the inner wall of tumor blood vessels. The stilbenes can kill tumor cells by cytotoxic T

lymphocyte antigen $CD8^+$, cut off the blood supply to tumor tissues at the same time, and thus cause rapid necrosis in solid tumors, together with inhibition on tumor metastasis, thereby, the killing effects on tumors are greatly improved. Thus, the stilbene compounds of the present invention can be used in the preparation of anti-tumor medicaments, and also used in combination with tumor immunotherapeutics to achieve synergistic anti-tumor effects. Among these stilbene compounds, the compounds with different structures may have different anti-tumor effects and thus can be used in the preparation of different anti-tumor drugs. In addition, the stilbene compounds of the present invention can inhibit the growth of ocular surface blood vessels and be used in the preparation of medicaments for treating various eye diseases. Collectively, the stilbene compounds of the present invention have good application prospects.

Obviously, based on the teachings of of the present invention, according to the common technical knowledge and the conventional means in the field, those skilled in the art may make various modifications, alternations, or changes without department from the above basic technical spirits.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. Flow chart of quantitative proteomics experiments.
Figure 2. HE staining results of human lung cancer 95D.
Figure 3. HE staining results of human liver cancer Bel7404.
Figure 4. PCNA immunohistochemical staining results of human lung cancer 95D.
Figure 5. PCNA immunohistochemical staining results of human liver cancer Bel7404.
Figure 6. The growth status of ocular surface blood vessels on the 10th day of administration (d10): A, the modeling group; B, the blank control group; C, treatment group 1; D, treatment group 2.

### Examples

The raw materials and equipments used in the specific examples of the present invention were known products obtained by purchasing commercially available products.

The structure of the stilbene compound according to the present invention is represented by formula (I):

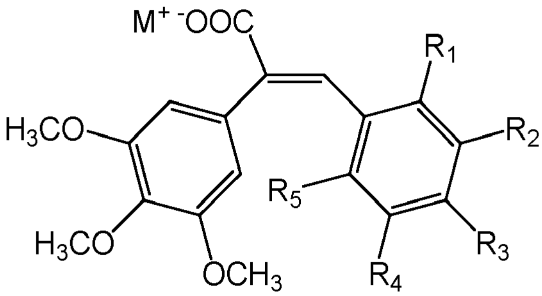

Formula (I)

wherein, M is selected from the group consisting of hydrogen, sodium, potassium, calcium, ammonium or an organic amine (e.g. amino acids, tert-butylamine, n-butylamine, and peptides); $R_1$, $R_2$, $R_3$, $R_4$, and $R_5$ are each selected from the group consisting of hydrogen, alkyl, hydroxy, methoxy, ethoxy, amino, fluorine, chlorine, or bromine.

The specific compounds of the present invention are shown in Table 1:

Table 1. Specific structures of the compounds according to the present invention.

| Compound | $R_1$ | $R_2$ | $R_3$ | $R_4$ | $R_5$ | M |
|---|---|---|---|---|---|---|
| 1 | -OH | -OH | $-OCH_3$ | -H | -H | -H |
| 2 | -OH | -OH | -OCH3 | -H | -H | N-methylglucamine |

(continued)

| Compound | $R_1$ | $R_2$ | $R_3$ | $R_4$ | $R_5$ | M |
|---|---|---|---|---|---|---|
| 3 | -H | $-OCH_3$ | -H | $-OCH_3$ | -H | Na |
| 4 | -H | -F | $-OCH_3$ | -H | -H | pyrazine |
| 5 | -H | -OH | $-OCH_3$ | -Cl | -H | alanine |
| 6 | -H | -OH | $-OCH_3$ | -H | -H | n-butylamine |
| 7 | -H | $-OCH_3$ | -OH | -H | -H | lysine |
| 8 | -H | -OH | $-OCH_3$ | -F | -H | tert-butylamine |
| 9 | -F | -OH | $-OCH_3$ | -H | -H | n-butylamine |
| 10 | -H | $-OCH_3$ | -F | $-OCH_3$ | -H | K |
| 11 | -H | -OH | $-OCH_3$ | -OH | -H | n-butylamine |
| 12 | $-OCH_3$ | -OH | $-OCH_3$ | -H | -H | lysine |
| 13 | -H | -OH | $-OCH_2CH_3$ | -H | -H | lysine |

**Example 1. Preparation of compound 1 according to the present invention**

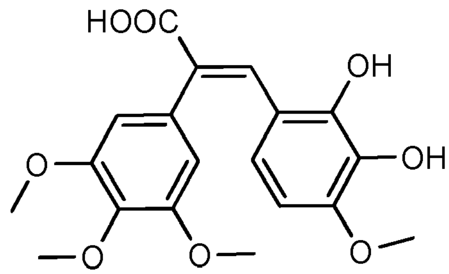

Compound **1**

**Preparation method:** To a 50 mL three necked flask, were added 2.26 g (0.010 mol) of 3,4,5-trimethoxyphenylacetic acid and 2.02 g (0.012 mol) of 2,3-dihydroxy-4-methoxybenzaldehyde, and then 3.2 mL of acetic anhydride and 1.6 mL of triethylamine were added. The mixture was shaken well, and then the reaction system was equipped with a magnetic stirrer, fitted with a reflux condenser, purged with nitrogen for protection, heated in an oil bath to 150 °C, and reacted for 5 h at this temperature. The reaction solution was cooled to 80 °C. The reflux condenser was replaced with a dropping funnel, and then 8 ml of hydrochloric acid solution was added dropwise. Then, the reaction mixture was allowed to react at 80 °C for 1 h, and then cooled to room temperature, to which was added 2 mL of 40% NaOH solution. The reaction mixture was allowed to react for additional 1 h, and then 1.8 mL of concentrated hydrochloric acid was added dropwise. After the solid precipitated, the reaction solution was further stirred and reacted for 1 h, and filtered. The filter cake was washed with cold absolute ethanol, and dried under reduced pressure, to obtain 2.17 g of off-white crystalline solids (compound **1**) (yield: 57.7%). $^1H$ NMR (400 MHz, DMSO) $\delta$3.672 (d, 9H, 3',4',5'-$OCH_3$), 3.821 (d, 3H, 4"-$OCH_3$), 5.265 (s, 1H, 2"-OH), 5.318 (s, 1H, 3"-OH), 6.273 (s, 1H, 2'-H), 6.348 (d, 1H, 6'-H), 6.386 (d, 1H, 5"-H), 7.415 (s, 1H, 3-H), 10.982 (s, 1H, CO-OH); $^{13}C$ NMR (100 MHz, DMSO) $\delta$57.692, 57.826, 62.837, 109.313, 112.713, 118.627, 125.392, 129.883, 137.423, 138.153, 138.232, 138.772, 146.391, 149.167, 154.482, 178.277, 16 carbon signals in total.

**Example 2. Preparation of compound 2 according to the present invention**

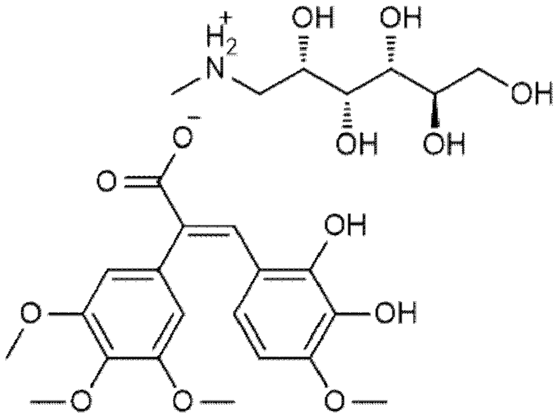

Compound **2**

**Preparation method:** To a 10 mL three necked flask, were added 1.82 g (4.84 mmol) of (E)-3-(2,3-dihydroxy-4-methoxyphenyl)-2-(3,4,5-trimethoxyphenyl)acrylic acid (compound **1**) and 0.99 g (5.08 mmol) of N-methylglucamine, and then 15 mL of ethanol was added. The reaction was mixed well. The flask was fitted with a magnetic stirrer. The reaction mixture was heated to 60 °C in a water bath, and after the reaction solution became clear, the solution was cooled to room temperature. After the solid precipitated, the reaction solution was further stirred for 1 h, and then filtered. The filter cake was washed with cold absolute ethanol, and dried under reduced pressure, to obtain 2.21 g of off-white crystalline solids (compound **2**) (yield: 80.0%). $^1$H NMR (400 MHz, DMSO) $\delta$2.791 (d, 3H, a-$CH_3$), 3.187 (d, 2H, a-$CH_2$), 3.297 (d, 1H, a-CH), 3.365 (d, 1H, a-CH), 3.382 (d, 1H, a-CH), 3.516 (d, 2H, a-$CH_2$), 3.683 (d, 4H, a-OH), 3.698 (d, 1H, a-OH), 3.713 (d, 9H, 3',4',5'-$OCH_3$), 3.796 (d, 3H, 4"-$OCH_3$), 5.159 (s, 1H, 2"-OH), 5.435 (s, 1H, 3"-OH), 6.368 (s, 1H, 2'-H), 6.521 (d, 1H, 6'-H), 6.472 (d, 1H, 5"-H), 7.477 (s, 1H, 3-H); $^{13}$C NMR (100 MHz, DMSO) $\delta$34.286, 51.217, 57.692, 57.826, 62.837, 63.295, 68.786, 71.198, 73.136, 73.982, 109.313, 112.713, 118.627, 125.392, 129.883, 137.423, 138.153, 138.232, 138.772, 146.391, 149.167, 154.482, 178.277, 23 carbon signals in total.

**Example 3. Preparation of compound 3 according to the present invention**

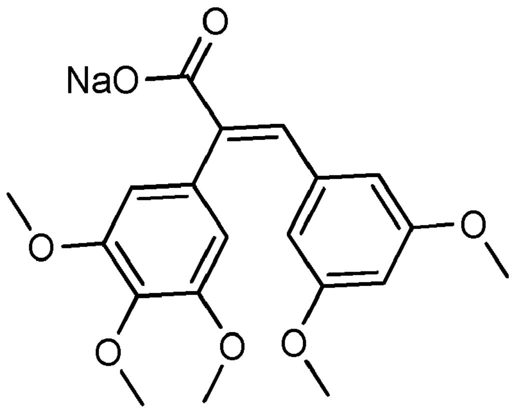

Compound **3**

**Preparation method:** To a 50 mL three necked flask, were added 2.26 g (0.010 mol) of 3,4,5-trimethoxyphenylacetic acid and 1.99 g (0.012 mol) of 3,5-dimethoxybenzaldehyde, and then 3.2 mL of acetic anhydride and 1.6 mL of triethylamine were added. The mixture was mixed well. The flask was equipped with a magnetic stirrer, fitted with a reflux condenser, and purged with nitrogen for protection. The reaction solution was heated in an oil bath to 150 °C, and then reacted for 5 h at this temperature. Subsequently, the reaction solution was cooled to 80 °C. The reflux condenser was replaced with a dropping funnel, and 8 ml of hydrochloric acid solution was added dropwise. The reaction mixture was allowed to react at 80 °C for 1 h, and then the reaction solution was cooled to room temperature, to which was

added 2 mL of 40% NaOH solution. The reaction mixture was allowed to react for additional 1 h, and then 1.8 mL of concentrated hydrochloric acid was added dropwise. After the solid precipitated, the reaction solution was further stirred and reacted for 1 h, and filtered. The filter cake was washed with cold absolute ethanol, and dried under reduced pressure, to obtain 2.25 g of (E)-3-(3,5-dimethoxyphenyl)-2-(3,4,5-trimethoxyphenyl)acrylic acid as off-white crystalline solids (yield: 60.1%).

To a 10 mL three necked flask, were added 1.81 g (4.84 mmol) of (E)-3-(3,5-dimethoxyphenyl)-2-(3,4,5-trimethoxyphenyl)acrylic acid, 10 mL of absolute ethanol, and 0.5 mL of 40% NaOH solution, and the reaction was mixed well. The flask was fitted with a magnetic stirrer and stirred. After the solid precipitated, the reaction solution was further stirred for 1 h, and then filtered. The filter cake was washed with cold absolute ethanol, and dried under reduced pressure, to obtain 1.62 g of off-white crystalline solids (compound 3) (yield: 84.4%). $^1$H NMR (400 MHz, DMSO) $\delta$ 3.727 (d, 9H, 3',4',5'-$OCH_3$), 3.869 (d, 6H, 3",5"-$OCH_3$), 6.174 (d, 1H, 4"-H), 6.273 (s, 1H, 2'-H), 6.348 (d, 1H, 6'-H), 6.386 (d, 1H, 5"-H), 6.723 (d, 2H, 2",5"-H)), 7.329 (s, 1H, 3-H); $^{13}$C NMR (100 MHz, DMSO) $\delta$ 55.823, 57.314, 62.729, 108.476, 113.626, 117.196, 126.181, 128.347, 136.973, 137.826, 137.951, 137.865, 147.127, 148.331, 154.672, 171.323, 16 carbon signals in total.

**Example 4. Preparation of compound 4 according to the present invention**

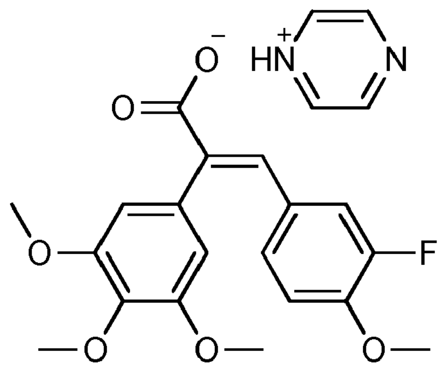

Compound **4**

**Preparation method:** To a 50 mL three necked flask, were added 2.26 g (0.010 mol) of 3,4,5-trimethoxyphenylacetic acid and 1.85 g (0.012 mol) of 3-fluoro-4-methoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and reacted for 5 h at this temperature. After that, the reaction solution was cooled to 80 °C. The reflux condenser was replaced with a dropping funnel, and then 8 ml of hydrochloric acid solution (1+5) was added dropwise. The reaction mixture was allowed to react at 80 °C for 1 h, and then the reaction solution was cooled to room temperature, to which was added 2 mL of 40% NaOH solution. The reaction mixture was allowed to react for additional 1 h, to which was then added 1.8 mL of concentrated hydrochloric acid dropwise. After the solid precipitated, the reaction solution was further stirred and reacted for 1 h, and filtered. The filter cake was washed with cold absolute ethanol, and dried under reduced pressure, to obtain 2.25 g of (E)-3-(3-fluoro-4-methoxyphenyl)-2-(3,4,5-trimethoxyphenyl)acrylic acid as off-white crystalline solids (yield: 59.3%).

To a 10 mL three necked flask, were added 1.75 g (4.84 mmol) of (E)-3-(3-fluoro-4-methoxyphenyl)-2-(3,4,5-trimethoxyphenyl)acrylic acid, 10 mL of absolute ethanol, and 0.41 g of pyrazine, and the reaction was mixed well. The flask was fitted with a magnetic stirrer and stirred. After the solid precipitated, the reaction solution was further stirred for 1 h, and then filtered. The filter cake was washed with cold absolute ethanol, and dried under reduced pressure, to obtain 1.76 g of off-white crystalline solids (compound **4**) (yield: 82.2%). $^1$H NMR (400 MHz, DMSO) $\delta$ 3.612 (d, 9H, 3',4',5'-$OCH_3$), 3.824 (d, 3H, 4"-$OCH_3$), 6.287 (s 2H, 2', 6'-H), 6.413 (s, 1H, 2"-H), 6.578 (d, 1H, 6"-H), 6.612 (d, 1H, 5"-H), 9.583 (s 2H C-H), 9.689 (s, 2H, C-H), 9.823 (s, 1H, 3-H); $^{13}$C NMR (100 MHz, DMSO) $\delta$ 56.276, 57.327, 63.196, 108.893, 113.746, 117.845, 125.637, 130.762, 138.524, 138.267, 139.241, 139.482, 142.158, 146.783, 148.298, 152.930, 174.128, 17 carbon signals in total.

**Example 5. Preparation of compound 5 according to the present invention**

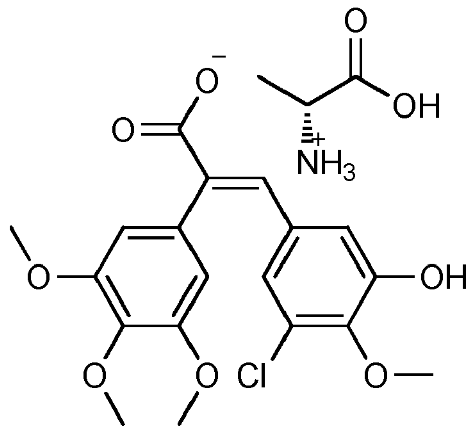

Compound **5**

**Preparation method:** To a 50 mL three necked flask, were added 2.26 g (0.010 mol) of 3,4,5-trimethoxyphenylacetic acid and 2.24 g (0.012 mol) of 3-chloro-5-hydroxy-4-methoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. Then, the reaction solution was heated in an oil bath to 150 °C, and reacted for 5 h at this temperature. After that, the reaction solution was cooled to 80 °C. The reflux condenser was replaced with a dropping funnel, and 8 ml of HCl solution was added dropwise. The reaction mixture was allowed to react at 80 °C for 1 h, and then the reaction solution was cooled to room temperature, to which was added 2 mL of 40% NaOH solution. The reaction mixture was allowed to react for additional 1 h, and then 1.8 mL of concentrated hydrochloric acid was added dropwise. After the solid precipitated, the reaction solution was further stirred and reacted for 1 h, and filtered. The filter cake was washed with cold absolute ethanol, and dried under reduced pressure, to obtain 2.13 g of (E)-3-(3-chloro-5-hydroxy-4-methoxyphenyl)-2-(3,4,5-trimethoxyphenyl) acrylic acid as off-white crystalline solids (yield: 53.9%).

To a 10 mL three necked flask, were added 1.75 g (4.84 mmol) of (E)-3-(3-chloro-5-hydroxy-4-methoxyphenyl)-2-(3,4,5-trimethoxyphenyl)acrylic acid, 0.45 g (5.08 mmol) of alanine, and 10 mL of absolute ethanol, and the reaction was mixed well. The flask was fitted with a magnetic stirrer, and the reaction solution was stirred. After the solid precipitated, the reaction solution was further stirred for 1 h, and then filtered. The filter cake was washed with cold absolute ethanol, and dried under reduced pressure, to obtain 1.42 g of off-white crystalline solids (compound **5**) (yield: 60.6%). $[M+H]^+$: 443.16. $^1$H-NMR ($CDCl_3$, $\delta$ (ppm)): 9.972 (2H), 9.712 (2H), 7.326 (1H), 6.887 (1H), 6.865 (1H), 6.853 (1H), 3.812 (3H), 3.924 (9H), 6.615 (2H).

**Example 6. Preparation of compound 6 according to the present invention**

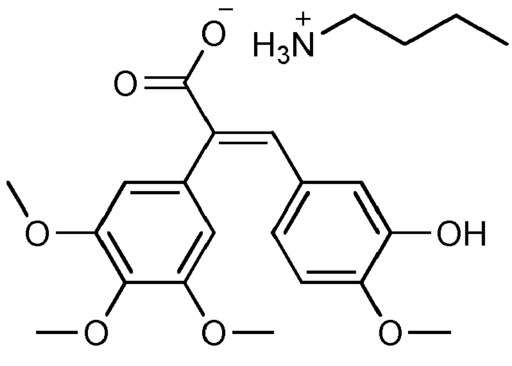

Compound **6**

**Preparation method:** To a 50 mL three necked flask, were added 2.26 g (0.010 mol) of 3,4,5-trimethoxyphenylacetic acid and 1.83 g (0.012 mol) of isovanillin, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and reacted for 5 h at this temperature. After that, the reaction solution was cooled to 80 °C. The reflux condenser was changed to a dropping funnel, and then 8 mL of hydrochloric acid solution was added dropwise. The reaction mixture was allowed to react at 80 °C for 1 h, and then the reaction solution was cooled to room temperature, to which was added 12 mL of 40% NaOH solution. The reaction mixture was allowed to react for additional 1 h, and then 10 mL of concentrated hydrochloric acid was added dropwise. After the solid precipitated, the reaction solution was further stirred and reacted for 1 h, and filtered. The filter cake was washed with cold absolute ethanol, and dried under reduced pressure, to obtain 2.56 g of (E)-3-(3-hydroxy-4-methoxyphenyl)-2-(3,4,5-trimethoxyphenyl)acrylic acid as off-white crystalline solids (yield: 71.0%).

To a 10 mL three necked flask, were added 1.74 g (4.84 mmol) of (E)-3-(3-hydroxy-4-methoxyphenyl)-2-(3,4,5-trimethoxyphenyl)acrylic acid, 0.37 g (5.08 mmol) of n-butylamine, and 10 mL of absolute ethanol, and the reaction was mixed well. The flask was fitted with a magnetic stirrer, to stir the reaction solution. After the solid precipitated, the reaction solution was further stirred for 1 h, and then filtered. The filter cake was washed with cold absolute ethanol, and dried under reduced pressure, to obtain 1.68 g of off-white crystalline solids (compound **6**) (yield: 80.1%). $^1H$ NMR (400 MHz, DMSO) $\delta$ 0.832 (d, 3H, a-$CH_3$),1.329 (d, 2H b-$CH_3$),1.546 (d, 2H, C-$CH_2$),2.914 (d, 2H, d-$CH_3$), 3.593 (d, 9H, 3',4',5'-$OCH_3$), 3.749 (d, 3H, 4"-$OCH_3$), 6.375 (s, 2H, 2'-H), 6.386 (s, 1H, 2"-H), 6.462 (d, 1H, 6"-H), 6.499 (d, 1H, 5"-H), 7.346 (s, 1H, 3-H); $^{13}C$ NMR (100 MHz, DMSO) $\delta$ 15.103, 21.343, 31.101, 41.526, 57.788, 58.186, 63.341, 109.224, 113.626, 118.900, 125.518, 130.918, 137.718, 137.812, 138.175, 139.291, 146.588, 149.858, 154.930, 178.099, 20 carbon signals in total.

**Example 7. Preparation of compound 7 according to the present invention**

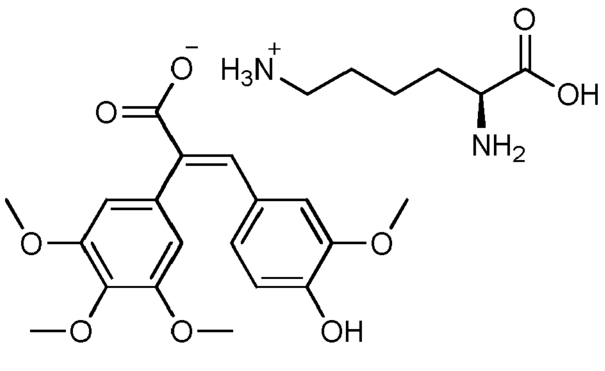

Compound 7

**Preparation method:** To a 50 mL three necked flask, were added 2.26 g (0.010 mol) of 3,4,5-trimethoxyphenylacetic acid and 1.83 g (0.012 mol) of vanillin, and then 3.2 mL of acetic anhydride and 1.6 mL of triethylamine were

added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of hydrochloric acid solution was added dropwise. The reaction mixture was allowed to react for 1 h at 80 °C, and then cooled to room temperature, to which was added 2 mL of 40% NaOH solution. The reaction mixture was reacted for additional 1 h, and then 1.8 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.74 g of (E)-3-(4-hydroxy-3-methoxyphenyl)-2-(3,4,5-trimethoxyphenyl)acrylic acid as off-white crystalline solids (yield: 76.0%).

To a 10 mL three necked flask, were added 1.74 g (4.84 mmol) of (E)-3-(4-hydroxy-3-methoxyphenyl)-2-(3,4,5-trimethoxyphenyl)acrylic acid, 0.74 g (5.08 mmol) of lysine, and 15 mL of absolute ethanol, and the reaction was mixed well. The flask was fitted with a magnetic stirrer. The reaction solution was heated to 60 °C in a water bath, and after the solution became clear, it was cooled to room temperature. After the solid precipitated, the reaction solution was further stirred for 1 h, and then filtered. The filter cake was washed with cold absolute ethanol, and dried under reduced pressure, to obtain 1.75 g of off-white crystalline solids (compound 7) (yield: 71.4%). $[M+H]^+$: 507.23. $^1$H-NMR ($CDCl_3$, $\delta$ (ppm)): 1.187 (2H), 1.742 (2H), 2.116 (2H), 3.281 (2H), 3.373 (1H), 5.095 (2H), 7.386 (1H), 3.659 (3H), 5.287 (1H), 7.137 (1H), 7.349 (1H), 3.876 (9H), 6.517 (2H).

**Example 8. Preparation of compound 8 according to the present invention**

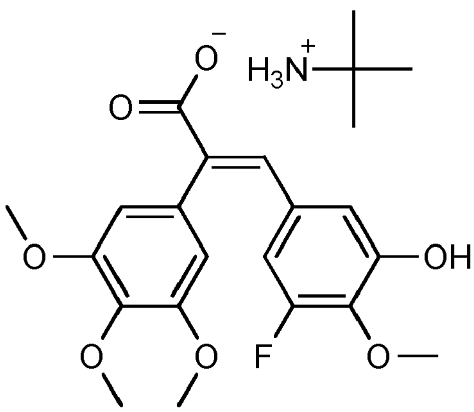

Compound **8**

**Preparation method:** To a 50 mL three necked flask, were added 2.26 g (0.010 mol) of 3,4,5-trimethoxyphenylacetic acid and 1.83 g (0.012 mol) of 5-fluoro-3-hydroxy-4-methoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of hydrochloric acid solution was added dropwise. The reaction mixture was allowed to react for 1 h at 80 °C, and then cooled to room temperature, to which was added 2 mL of 40% NaOH solution. The reaction mixture was reacted for additional 1 h, and then 1.8 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.45 g of (E)-3-(5-fluoro-3-hydroxy-4-methoxyphenyl)-2-(3,4,5-trimethoxyphenyl)acrylic acid as off-white crystalline solids (yield: 64.7%).

To a 10 mL three necked flask, were added 1.74 g (4.84 mmol) of (E)-3-(5-fluoro-3-hydroxy-4-methoxyphenyl)-2-(3,4,5-trimethoxyphenyl)acrylic acid, 0.37 g (5.08 mmol) of tert-butylamine, and 10 mL of absolute ethanol, and the reaction was mixed well. The flask was fitted with a magnetic stirrer. The reaction solution was stirred, and the solids precipitated. Then, the reaction solution was further stirred for 1 h, and filtered. The filter cake was washed with cold absolute ethanol, and dried under reduced pressure, to obtain 1.52 g of off-white crystalline solids (compound 8) (yield: 69.6%). $[M+H]^+$: 452.20. $^1$H-NMR ($CDCl_3$, $\delta$ (ppm)): 1.287 (9H), 3.635 (3H), 3.831 (9H), 6.228 (2H), 7.267 (1H), 7.338 (1H), 7.643 (1H).

**Example 9. Preparation of compound 9 according to the present invention**

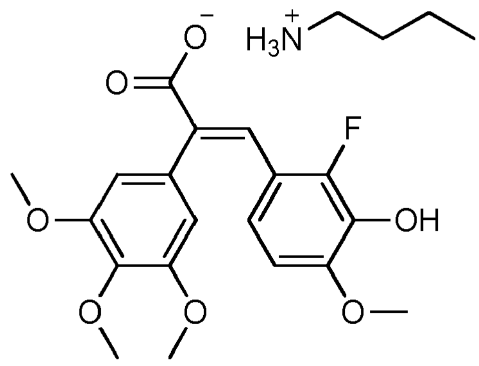

Compound **9**

**Preparation method:** To a 50 mL three necked flask, were added 2.26 g (0.010 mol) of 3,4,5-trimethoxyphenylacetic acid and 1.83 g (0.012 mol) of 2-fluoro-3-hydroxy-4-methoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of hydrochloric acid solution was added dropwise. The reaction mixture was allowed to react for 1 h at 80 °C, and then cooled to room temperature, to which was added 12 mL of 40% NaOH solution. The reaction mixture was reacted for additional 1 h, and then 10 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.56 g of (E)-3-(2-fluoro-3-hydroxy-4-methoxyphenyl)-2-(3,4,5-trimethoxyphenyl)acrylic acid as off-white crystalline solids (yield: 67.6%).

To a 10 mL three necked flask, were added 1.74 g (4.84 mmol) of (E)-3-(2-fluoro-3-hydroxy-4-methoxyphenyl)-2-(3,4,5-trimethoxyphenyl)acrylic acid, 0.37 g (5.08 mmol) of n-butylamine, and 10 mL of absolute ethanol, and the reaction was mixed well. The flask was fitted with a magnetic stirrer. The reaction solution was stirred. After the solids precipitated, the reaction solution was further stirred for 1 h, and filtered. The filter cake was washed with cold absolute ethanol, and dried under reduced pressure, to obtain 1.78 g of off-white crystalline solids (compound **9**) (yield: 81.5%). $[M+H]^+$: 452.20. $^1$H-NMR ($CDCl_3$, $\delta$ (ppm)): 0.912 (3H), 1.276 (2H), 2.314 (2H), 3.251 (2H), 3.586 (3H), 3.773 (9H), 6.643 (2H), 7.084 (1H), 7.237 (1H), 7.539 (1H).

**Example 10. Preparation of compound 10 according to the present invention**

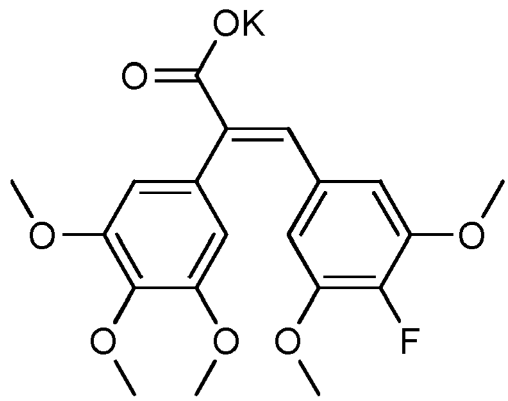

Compound **10**

**Preparation method:** To a 50 mL three necked flask, were added 2.26 g (0.010 mol) of 3,4,5-trimethoxyphenylacetic acid and 1.99 g (0.012 mol) of 4-fluoro-3,5-dimethoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of hydrochloric acid solution was added dropwise. The reaction mixture was allowed to react for 1 h at 80 °C, and then cooled to room temperature, to which was added 2 mL of 40% NaOH solution. The reaction mixture was reacted for additional 1 h, and then 1.8 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.47 g of (E)-3-(3,5-dimethoxyphenyl)-2-(3,4,5-trimethoxyphenyl)acrylic acid as off-white crystalline solids (yield: 62.9%).

To a 10 mL three necked flask, were added 1.81 g (4.84 mmol) of (E)-3-(3,5-dimethoxyphenyl)-2-(3,4,5-trimethoxyphenyl)acrylic acid, 10 mL of absolute ethanol, and 0.7 mL of 40% KOH solution, and the reaction was mixed well. The flask was fitted with a magnetic stirrer. The reaction solution was stirred. After the solids precipitated, the reaction solution was further stirred for 1 h, and filtered. The filter cake was washed with cold absolute ethanol, and dried under reduced pressure, to obtain 1.45 g of off-white crystalline solids (compound **10**) (yield: 69.6%). $[M+H]^+$: 432.08. $^1$H-NMR ($CDCl_3$, $\delta$(ppm)): 3.541 (3H), 3.682 (3H), 3.773 (9H), 6.327 (2H), 6.495 (1H), 6.676 (1H), 7.329 (1H).

**Example 11. Preparation of compound 11 according to the present invention**

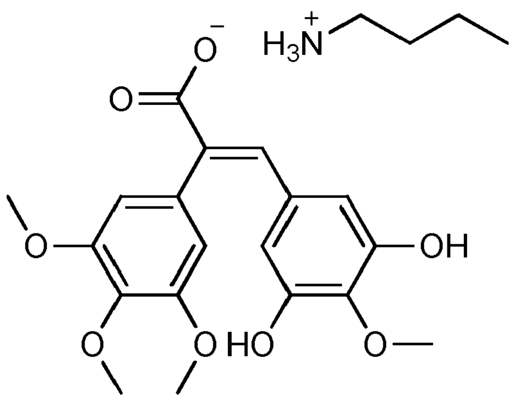

Compound **11**

**Preparation method:** To a 50 mL three necked flask, were added 2.26 g (0.010 mol) of 3,4,5-trimethoxyphenylacetic acid and 2.02 g (0.012 mol) of 3,5-dihydroxy-4-methoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of hydrochloric acid solution was added dropwise. The reaction mixture was allowed to react for 1 h at 80 °C, and then cooled to room temperature, to which was added 2 mL of 40% NaOH solution. The reaction mixture was reacted for additional 1 h, and then 1.8 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.14 g of (E)-3-(3,5-dihydroxy-4-methoxyphenyl)-2-(3,4,5-trimethoxyphenyl)acrylic acid as off-white crystalline solids (yield: 56.8%).

To a 10 mL three necked flask, were added 1.82 g (4.84 mmol) of (E)-3-(3,5-dihydroxy-4-methoxyphenyl)-2-(3,4,5-trimethoxyphenyl)acrylic acid, 0.37 g (5.08 mmol) of n-butylamine, and 10 mL of absolute ethanol, and the reaction was mixed well. The flask was fitted with a magnetic stirrer. The reaction solution was stirred. After the solids precipitated, the reaction solution was further stirred for 1 h, and filtered. The filter cake was washed with cold absolute ethanol, and dried under reduced pressure, to obtain 1.56 g of off-white crystalline solids (compound **11**) (yield: 71.7%). $[M+H]^+$: 450.21. $^1$H-NMR ($CDCl_3$, $\delta$(ppm)): 0.821 (3H), 1.327 (2H), 2.172 (2H), 3.319 (2H), 3.591 (3H), 3.836 (9H), 5.243 (2H), 6.385 (2H), 7.326 (2H), 7.613 (1H).

**Example 12. Preparation of compound 12 according to the present invention**

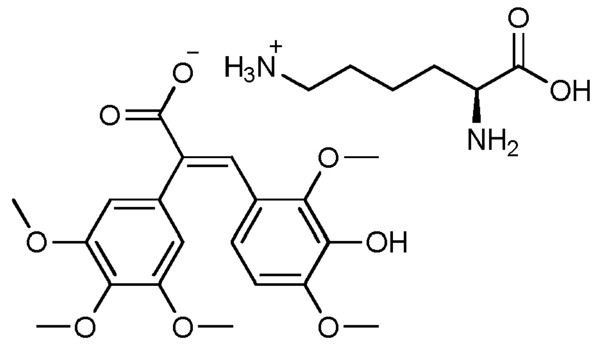

Compound **12**

**Preparation method:** To a 50 mL three necked flask, were added 2.26 g (0.010 mol) of 3,4,5-trimethoxyphenylacetic acid and 2.19 g (0.012 mol) of 3-hydroxy-2,4-dimethoxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of hydrochloric acid solution was added dropwise. The reaction mixture was allowed to react for 1 h at 80 °C, and then cooled to room temperature, to which was added 2 mL of 40% NaOH solution. The reaction mixture was reacted for additional 1 h, and then 1.8 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.38 g of (E)-3-(3-hydroxy-2,4-dimethoxyphenyl)-2-(3,4,5-trimethoxyphenyl)acrylic acid as off-white crystalline solids (yield: 61.0%).

To a 10 mL three necked flask, were added 1.89 g (4.84 mmol) of (E)-3-(3-hydroxy-2,4-dimethoxyphenyl)-2-(3,4,5-trimethoxyphenyl)acrylic acid and 0.74 g (5.08 mmol) of lysine, followed by addition of ethanol (15 mL), and then the reaction was mixed well. The flask was fitted with a magnetic stirrer. The reaction solution was heated to 60 °C in a water bath, and after the solution became clear, it was cooled to room temperature. After the solids precipitated, the reaction solution was further stirred for 1 h, and filtered. The filter cake was washed with cold absolute ethanol, and dried under reduced pressure, to obtain 1.85 g of off-white crystalline solids (compound **12**) (yield: 75.5%). $[M+H]^+$: 537.24. $^1$H-NMR ($CDCl_3$, $\delta$(ppm)): 1.183 (2H), 1.649 (2H), 2.173 (2H), 3.286 (2H), 3.513 (1H), 3.597 (3H), 3.876 (9H), 5.237 (2H), 5.459 (1H), 6.525 (2H), 7.193 (1H), 7.315 (1H), 7.613 (1H).

**Example 13. Preparation of compound 13 according to the present invention**

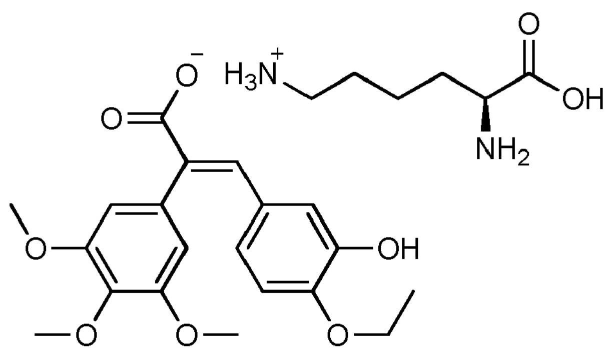

Compound **13**

**Preparation method:** To a 50 mL three necked flask, were added 2.26 g (0.010 mol) of 3,4,5-trimethoxyphenylacetic acid and 1.99 g (0.012 mol) of 4-ethoxy-3-hydroxybenzaldehyde, and then 3.2 mL of acetic anhydride and 1.6 mL of triethylamine were added. The mixture was mixed well. The flask was equipped with a magnetic stirrer and a reflux condenser, and purged with nitrogen for protection. The reaction solution was heated in an oil bath to 150 °C, and allowed to react for 5 h at this temperature. Subsequently, the reaction solution was cooled to 80 °C. The reflux condenser was changed to a dropping funnel, and then 8 mL of HCl solution was added dropwise. The reaction mixture was allowed to react for 1 h at 80 °C, and then cooled to room temperature, to which was added 2 mL of 40% NaOH solution. The reaction mixture was reacted for additional 1 h, and then 1.8 mL of concentrated HCl was added dropwise. After the solid precipitated, the reaction solution was further stirred and reacted for 1 h, and filtered. The filter cake was washed with cold absolute ethanol, and dried under reduced pressure, to obtain 2.02 g of (E)-3-(4-ethoxy-3-hydroxyphenyl)-2-(3,4,5-trimethoxyphenyl) acrylic acid as off-white crystalline solids (yield: 53.9%).

To a 10 mL three necked flask, were added 1.81 g (4.84 mmol) of (E)-3-(4-ethoxy-3-hydroxyphenyl)-2-(3,4,5-trimethoxyphenyl)acrylic acid and 0.74 g (5.08 mmol) of lysine, followed by addition of ethanol (15 mL), and then the reaction was mixed well. The flask was fitted with a magnetic stirrer. The reaction solution was heated to 60 °C in a water bath, and after the solution became clear, it was cooled to room temperature. After the solids precipitated, the reaction solution was further stirred for 1 h, and filtered. The filter cake was washed with cold absolute ethanol, and dried under reduced pressure, to obtain 2.05 g of off-white crystalline solids (compound **13**) (yield: 81.4%). $[M+H]^+$: 521.25. $^1$H-NMR ($CDCl_3$, $\delta$(ppm)): 1.187 (2H), 1.341 (3H), 1.843 (2H), 2.117 (2H), 3.415 (2H), 3.523 (1H), 3.786 (9H), 4.217 (2H), 5.302 (2H), 5.476 (1H), 6.558 (2H), 7.181 (1H), 7.328 (1H), 7.613 (1H).

**Example 14. Preparation of the compound according to the present invention and NaCl injection (0.9%)**

0.9% of the compound according to the present invention and NaCl injection was prepared for intravenous infusion:

**Formulation 1:**

| | |
|---|---|
| Compound **1** | 1 g |
| Citric acid | 1.0 g |
| Sodium citrate | 0.5 g |
| NaCl | 18 g |
| Injection water was added to | 2000 mL |

According to the general procedures of the injection, a total of 1000 injections (2 ml/injection) were prepared, each containing 1 mg of compound **1.**

**Formulation 2:**

| | |
|---|---|
| Compound **2** | 1500 g |
| NaCl | 180 g |
| Injection water was added to | 20000 mL |

According to the general procedures of the injection, a total of 1000 injections (20 mL/injection) were prepared, each containing 1.5 g of compound **2.**

**Formulation 3:**

| | |
|---|---|
| Compound **2** | 3000 g |
| NaCl | 9000 g |
| Injection water was added to | 1000000 mL |

According to the general procedures of the injection, a total of 1000 injections (1000 mL/injection) were prepared, each containing 3 g of compound **2.**

In order to improve the stability of the compound, when preparing the injection, the pharmaceutically acceptable common stabilizers, antioxidants, and pH regulators could also be added into the above formulations according to the conventional methods; for example, stabilizers such as cylodextrins inclusion compound; surfactants (anionic surfactants, cationic surfactants, zwitterion surfactants, non-ionic surfactants), etc; antioxidants such as sodium sulfite, sodium bisulfite, sodium metabisulfite, sodium thiosulfate, ascorbic acid, cysteine, etc; pH regulators such as citric acid, fumaric

acid, glutamic acid, L-aspartic acid, lactic acid, lactobionic acid, galacturonic acid, glucuronic acid, ascorbic acid, hydrochloric acid, acetic acid, etc.

**Example 15: Preparation of sterile powder injection containing compounds of the present invention**

**Formulation 1:**

| | |
|---|---|
| Compound **3** sterile powder | 1 g |
| NaCl sterile powder | 18 g |

According to the general procedures of sterile powder injection, a total of 1000 powder injections (2 mL/powder injection) were prepared, each containing 1 mg of compound **3.**

**Formulation 2:**

| | |
|---|---|
| Compound **3** sterile powder | 1500 g |
| NaCl sterile powder | 180 g |

According to the general procedures of sterile powder injection, a total of 1000 powder injections (20 mL/powder injection) were prepared, each containing 1.5 g of compound **3.**

**Formulation 3:**

| | |
|---|---|
| Compound **3** sterile powder | 3000 g |

According to the general procedures of sterile powder injection, a total of 1000 powder injections (5 mL/powder injection) were prepared, each containing 3 g of compound **3.**

**Example 16. Preparation of the compound according the present invention and glucose injection (5%)**

5% of the compound according to the present invention and glucose injection was prepared for intravenous infusion:

**Formulation 1:**

| | |
|---|---|
| Compound **5** | 1 g |
| Citric acid | 1.0 g |
| Sodium citrate | 0.5 g |
| Glucose | 100 g |
| Injection water was added to | 2000 mL |

According to the general procedures of the injection, a total of 1000 injections (2 mL/injection) were prepared, each containing 1 mg of compound **5.**

**Formulation 2:**

| | |
|---|---|
| Compound **5** | 1500 g |
| Glucose | 1000 g |
| Injection water was added to | 20000 mL |

According to the general procedures of the injection, a total of 1000 injections (20 mL/injection) were prepared, each containing 1.5 g of compound **5.**

**Formulation 3:**

| | |
|---|---|
| Compound **5** | 3000 g |
| Glucose | 10000 g |

| | |
|---|---|
| Injection water was added to | 1000000 mL |

According to the general procedures of the injection, a total of 1000 injections (1000 mL/injection) were prepared, each containing 3 g of compound **5.**

**Example 17: Preparation of tablets containing the compound of the present invention**

**Formulation:**

| | |
|---|---|
| Compound **6** | 1.00 g |
| Starch | 184.00 g |
| Polyvinyl pyrrolidone | 5.00 g |
| Magnesium stearate (120 mesh) | 10.00 g |
| Totalling | 200.00 g |

A total of 1000 tablets were prepared, each tablet containing 1 mg of compound **6.**

When preparing tablets, bulking agents can be selected from the group consisting of starch, dextrin, sugar powder, pregelatinized starch, lactose, glucose, microcrystalline cellulose, calcium carbonate, calcium sulfate, calcium bicarbonate, etc; adhesive agents can be selected from the group consisting of hydroxypropyl methyl cellulose, povidone, starch slurry, dextrin slurry, syrup, mucilage, sodium alginate, polyethylene glycol, peach gum, arabic gum, etc; disintegrating agents can be selected from the group consisting of croscarmellose sodium, cross-linked povidone, sodium carboxymethyl starch, hydroxypropyl starch, low substituted hydroxypropyl cellulose, citric acid, tartaric acid, anhydride, sodium bicarbonate, sodium carbonate, etc.

**Example 18: Preparation of capsules containing the compound of the present invention**

**Formulation**

| | |
|---|---|
| Compound **9** | 100.00 g |
| Starch | 85.00 g |
| Polyvinyl pyrrolidone | 5.00 g |
| Magnesium stearate (120 mesh) | 10.00 g |
| Totalling | 200.00 g |

A total of 1000 capsules were prepared, each capsule containing 100 mg of compound **9.**

When preparing capsules, bulking agents can be selected from the group consisting of starch, dextrin, sugar powder, pregelatinized starch, lactose, glucose, microcrystalline cellulose, calcium carbonate, calcium sulfate, calcium bicarbonate, etc; adhesive agents can be selected from the group consisting of hydroxypropyl methyl cellulose, povidone, starch slurry, dextrin slurry, syrup, mucilage, sodium alginate, polyethylene glycol, peach gum, arabic gum, etc;

The beneficial effects of the present invention were demonstrated with reference to the following specific Experimental examples.

**Experimental example 1: Study on the enrichment of cytotoxic T lymphocyte antigen $CD8^+$ by the compound of the present invention in the Lewis non-small cell lung cancer model of mice**

LLC (mouse lung cancer cells) were a mouse Lewis lung cancer cells.

The modeling method for LLC cell lung cancer in mice was as follows:

LLC cells were cultured in the general medium containing 10% fetal bovine serum and 100 μg/mL of double antibody (penicillin+streptomycin), and then cultivated in a 37 °C, 5% $CO_2$ cell incubator for routine incubation. LLC cells, that had grown to logarithmic phase, were digested with 0.25% trypsin (containing EDTA) to form a cell suspension, which was centrifuged at 1000 rpm/min for 3 min. The supernatant was discarded, and cell precipitates were collected, that were resuspended in culture medium without serum and double antibody. The cell suspension was further centrifuged to adjust the volume under the same conditions with the aid of cell counting, so as to prepare a cell suspension at a concentration of $1 \times 10^7$/μL. 200 μL of cell suspension was injected into the right abdomen of each mouse, to complete

the inoculation of tumor cells. The tumor was allowed to grew about 10 days, and the volume reached around 100 $mm^3$, before the mice were treated with test compounds.

Drug preparation: compounds **1-13** of the present invention were respectivle prepared into an injection with injection water.

Experimental methods: The model mice with LLC cell lung cancer were treated by intraperitoneal administration of 400 mg/kg injection containing the compound of the present invention. The model mice were divided into a negative control group (model mice only receiving equal volume of injection water by intraperitoneal administration) and treatment groups (model mice receiving the injection prepared with the compound of the present invention by intraperitoneal administration). The intraperitoneal injection regimen was carried out by injecting once a day at the pre-determined time. Before administration, the mice were weighed, and then treated with the injection at a dosage of 10 mL/kg. After successive administration for 10 days, that is, on the 10th day after receiving treatment, the mice were sacrificed, and the subcutaneous tumors were stripped to detect the ratio of cytotoxic T lymphocyte antigen $CD8^+$ in tumor tissues of the negative group and the treatment group by flow cytometry.

$CD8^+$ tumor-infiltrating lymphocytes in tumor tissues were detected by flow cytometry. The specific steps were as follows:

The mice were sacrificed, and then the abdominal epidermis of the mice was cut open using ophthalmic scissors, to fully expose the subcutaneous tumors. The subcutaneous tumor tissues on the right side of the mouse's abdomen were gradually peeled off with tweezers and ophthalmic scissors. After rinsing with PBS, the stripped tumor tissues were cut into about 1 mm using ophthalmic scissors, and then placed in 3 mL of PBS buffer containing 100 $\mu$g/mL of hyaluronidase. A centrifuge tube containing a dispersed liquid was placed in a water bath and kept at 37 °C for 20 min. A pipette was used to blow and shake the digestive tumor tissues every 5 minutes to fully digest. The dispersed liquid containing undigested tumor tissues was filtered through a 70 $\mu$m stainless steel cell screen, and the remaining undigested tumor tissues were ground using a push rod of 20 mL syringe on 70 $\mu$m cell screen and rinsed 5 times with 1 mL of PBS while grinding. The cell suspension passing the cell screen was transferred to a 15 mL centrifuge tube, and centrifuged at 4000 rpm for 3 min. Then, cell precipitates were collected, to which was added 2 mL of red cell lysis buffer, and rested for 10 min, followed by centrifuging at 4000 rpm for 3 min. After washing with PBS, the cells were resuspended by adding 2-3 mL of PBS.

An appropriate amount of cell suspension was used as the sample for flow cytometry, and after counting, the cell suspension was adjusted to have a concentration of $5 \times 10^6$/mL. 100 $\mu$L of cell suspension was added to a test tube with a pipettor. FITC anti-mouse CD3, PE-Cy5 anti-mouse CD8, and PE/Cyanine7 anti-mouse CD45 were respectively added to each test tube, in which the first two were simultaneously added to a test tube. After adding fluorescent antibodies, the test tube was placed on ice and incubated in dark for 20 min. After adding 2 mL of sterilized PBS, the test tube was centrifuged at 1000 rpm for 5 min, and the supernatant was discarded. 0.5 mL of sterilized PBS was added to resuspend the cells, and a total cell count was $1 \times 10^6$ by detection with flow cytometry. The results were analyzed using BD-FACSDiVa.

Comparing the ratio of cytotoxic T lymphocytes containing $CD8^+$ antigen in $CD45^+$, $CD3^+$, and $CD8^+$ cells:

The IHC Profiler plugin of ImageJ was used to process the tumor immunohistochemical microscopic image. The color deconvolution of the staining image was performed to separate the blue negative area and the brown positive area, and then the percentage of the positive area in the total area of the staining region was obtained.

The effect of drugs on the distribution of $CD8^+$ cytotoxic T lymphocytes (CTL) in tumor tissues and their cytotoxic effects on tumor cells were detected by immunohistochemistry. The specific procedures were as follows:

After 10 days of treatment, the mice were killed, the abdominal epidermis of the mice was cut open with ophthalmic scissors, and the tumor was removed and placed in formalin for fixation. After completion of fixation, the tumor tissues were embeded with paraffin, and two consecutive slices were cut off, which were transferred on a glass slide and dried (60 °C, 1 h). After adding the primary antibody respectively, the slide was allowed to rest overnight at 4 °C, followed by adding the second antibody dropwise. After staining with 4-aminodiphenyl (DAB), the slide was re-stained with haematoxylin, dehydrated, and covered after becoming transparent.

The entire slide image was captured at a 20× magnification, and then the stained section image was processed in ImageJ to distinguish between blue negative areas and brown positive areas, and the percentage of positive areas in the total section area was calculated.

The research results are shown in Table 2.

Table 2. The effect of the compound according to the present invention on the enrichment of $CD8^+$ in tumor tissues and the results of its distribution area ratio.

| Compound | The ratio of $CD8^+$ in total immune cells of tumor tissues (%) | The ratio of distribution area in tumor tissues (%) |
|---|---|---|
| **1** | 0.0605 ± 0.0124 | 6.265± 0.3673 |
| **2** | 0.0823 ± 0.0112 | 7.147± 0.7528 |
| **3** | 0.0762 ± 0.0103 | 6.731± 0.5692 |
| **4** | 0.0787 ± 0.0122 | 6.872± 0.7633 |
| **5** | 0.0833 ± 0.0107 | 7.438± 0.7610 |
| **6** | 0.0814 ± 0.0118 | 7.249± 0.6689 |
| **7** | 0.0774 ± 0.0115 | 6.653± 0.7791 |
| **8** | 0.0735 ± 0.0127 | 6.543± 0.7256 |
| **9** | 0.0753 ± 0.0123 | 6.258± 0.6573 |
| **10** | 0.0623 ± 0.0114 | 5.847± 0.6349 |
| **11** | 0.0653 ± 0.0122 | 5.735± 0.5952 |
| **12** | 0.0753 ± 0.0103 | 5.883± 0.6723 |
| **13** | 0.0723 ± 0.0126 | 6.547± 0.5843 |
| Blank control | 0.0417 ± 0.0108 | 2.125± 0.2404 |

Based on the experimental results, the compound of the present invention had a dual effect of enriching cytotoxic T lymphocyte antigen $CD8^+$ in tumor tissues and targeting the destruction of the inner wall of tumor blood vessels. The compound could greatly increase the enrichment ratio and distribution area of cytotoxic T lymphocyte antigen $CD8^+$ in tumor tissues, and while killing tumor cells by cytotoxic T lymphocyte antigen $CD8^+$, it cuted off the blood supply to the tumor tissues, causing rapid necrosis within solid tumors.

**Experimental example 2. Effect of the compound according to the present invention on the adhesion protein of nonfusion human umbilical vein endothelial cell (HUVEC)**

Fusion HUVECs: HUVECs in the logarithmic growth phase were inoculated into a 96-well culture plate at $8\times10^4$/mL and 100 μL/well (i.e. 8000 cells/well). The cells were cultured in the medium containing 10% fetal bovine serum, and then placed in a 37 °C, 5% $CO_2$ incubator for further incubation. After 24 h, the cells were treated with the test compound. The test compound was diluted with PBS(-) and added to each well (10 μL/well), 3 multiple wells for each concentration. The plate was cultivated for 24 h in the incubator, and then 10 μL of CCK-8 solution was added to each well, respectively, and during this process, it should be careful not to produce bubbles. Subsequently, the plate was incubated for additional about 2.5 h in the incubator, followed by measuring the absorbance. The OD value was obtained at a wavelength of 450 nm using an MK-2 fully automatic enzyme-linked immunosorbent assay system, and thus the $IC_{50}$ value was calculated.

Nonfusion HUVECs: HUVECs in the logarithmic growth phase were inoculated in a 96-well plate at $2\times10^4$/mL and 100 μL/well (i.e. 2000 cells/well). The cells were cultured in the medium containing 10% fetal bovine serum, and then placed in a 37 °C, 5% $CO_2$ incubator for further incubation. After 24 h, the cells were treated with the test compound. The test compound was diluted with PBS(-) and added to each well (10 μL/well), 3 multiple wells for each concentration. After the plate was transferred to the incubator and cultivated for 24 h, 10 μL of CCK-8 solution was added to each well, and during this process, close attention should be paid to avoid the generation of air bubbles. After the plate was incubated for additional about 2.5 h in the incubator, the absorbance can be measured. The OD value was tested at a wavelength of 450 nm using an MK-2 fully automatic enzyme-linked immunosorbent assay system, and thus the $IC_{50}$ value was calculated.

Nonfusion human umbilical vein endothelial cells (HUVEC) were used to simulate new tumor blood vessels, while confluent HUVECs were used to simulate normal blood vessels, to construct experimental models. Two different HUVECs were treated with the compound at the same dosage of 10 μM, to simulate the fusion cell state, and proteome samples were prepared. After preparation of peptide segments, the segements were labelled with TMT, and a quantitative

proteomics analysis was carried out. The efficiency of TMT labeling was higher than 99.4%, and a total of 28372 peptide segments from 4934 proteins were characterized using a strict 1% FDR limit. Among them, 4909 quantifiable proteins were identified, indicating reliable quality. The nonfusion treatment groups and the fusion treatment groups were compared with their respective control groups, and the control group was used as a reference, with a significant difference of more than 1.5 times or less than 0.67 times as the protein score standard.

The total cell proteins of the samples in nonfusion cell groups and fusion cell groups were extracted separately, and 2 μg of protein samples were subjected to quality analysis. The quantitative proteomics experiments were carried out, and the protein concentration assay was performed by quantitative grayscale measurement. The specific flowchart is shown in Figure 1.

**Data analysis:** The human protein database (Scientific name: *Homo sapiens,* 9606) was downloaded from UniProt. MaxQuant (v 1.5.5.1) search engine was used to search for the database. A total of 4934 proteins (4909 quantitative proteins) and 28372 peptide segments were identified by TMT quantitative proteomics experiments, with a labeling efficiency of more than 99%, and the complete digestion occupied 86.3%.

The proteomic assay results of the compound according to the present invention on nonfusion human umbilical vein endothelial cells (HUVEC) are shown in Table 3.

Table 3. The effect of compounds according to the present invention on proteins.

| Compound | Differentially expressed proteins | Up-regulation | Down-regulation |
|---|---|---|---|
| **1** | 323 | 7 | 296 |
| **2** | 298 | 10 | 288 |
| **3** | 318 | 6 | 312 |
| **4** | 307 | 5 | 302 |
| **5** | 304 | 7 | 297 |
| **6** | 312 | 5 | 307 |
| **7** | 289 | 8 | 281 |
| **8** | 317 | 6 | 311 |
| **9** | 296 | 5 | 291 |
| **10** | 315 | 6 | 309 |
| **11** | 317 | 11 | 306 |
| **12** | 322 | 8 | 314 |
| **13** | 319 | 7 | 312 |
| Blank | 0 | 0 | 0 |
| control | | | |

GO enrichment analysis was performed on differential proteins, and nonfusion differential proteins were mainly involved in regulating intercellular adhesion. It indicated that the above compounds targetted to destroy the adhesion function of nonfusion human umbilical vein endothelial cells (HUVECs), made the endothelial cells detach from the vascular base, thus causing nonfusion vascular inflammatory reactions and further blocking the blood vessels.

**Experimental example 3: The efficacy of compounds on human lung cancer A549 transplanted tumors in nude mice**

In this experiment, a human lung cancer A549 model transplanted into nude mice was selected to study the anti-tumor effect of test compounds. The mice were treated at a dosage of 400 mg/kg by gavage for 14 days, and the tumor inhibition rate against human lung cancer A549 was measured.

Preparation: The compound was prepared as the solution at the desired concentration with distilled water, prior to administration.

Animal strain: BALB/C nude mice (SPF grade), weight: 19-21 g, gender: male.

Number of animals in each treatment group: 8; Number of animals in the control group: >12.

**Experimental method:**

Lung cancer A549 tumor blocks with good growth were chosen and cut into uniform small pieces of 3 mm under sterile conditions, and then subcutaneously inoculated into the right armpit of each mouse with a cannula. These mice were divided into 9 groups, 8 mice for each treatment group, while more than 12 mice were included in the control group.

14 days after inoculation, the average volume of the tumor mass was about 100 $mm^3$. The animals were regrouped based on the size of the tumor, and the mice with tumors that were too large and too small were eliminated. For each group, the average volume of tumors was basically the same, and then the mice were treated with test compounds. The mice in the treatment groups were administered once a day by gavage for 14 successive days, at a dosage of 0.5 mL/20 g body weight; the mice in the control group were given the same volume of physiological saline. From the 14th day of inoculation, the long diameter a (mm) of the tumor, together with the perpendicular short diameter b (mm), was measured using a digital electronic caliper, twice a week. The calculation formula of tumor volume is: $TV = ab^2/2$, and the formula for calculating the relative tumor volume is RTV = Vt/Vo, wherein Vo denotes the tumor volume measured when the mice were divided into groups (i.e. d1), and Vt is the tumor volume from each measurement.

The result determination is based on the following formula:

$$\text{Tumor inhibitory rate\%} = \frac{\text{The average RTV of the control group - The average RTV of the treatment group}}{\text{The average RTV of the control group}} \times 100\%$$

The experimental results are shown in Table 4:

Table 4. The inhibitory effect of compounds on the tumor mass of lung cancer A549.

| Group | Dosage (mg/kg) | Dosage regimen | Animal numbers Beginning | Animal numbers end | RTV (d22) | Tumor inhibitory rate% |
|---|---|---|---|---|---|---|
| Control | | | 16 | 16 | 11.75±1.38 | |
| Compound **1** | 400 | ig×14 | 8 | 8 | 4.27±0.48** | 63.65## |
| Compound **3** | 400 | ig×14 | 8 | 8 | 4.52±0.76** | 61.53## |
| Compound **4** | 400 | ig×14 | 8 | 8 | 5.23±1.43** | 55.48## |
| Compound **5** | 400 | ig×14 | 8 | 8 | 5.73±1.25** | 51.23## |
| Compound **6** | 400 | ig×14 | 8 | 8 | 2.51±0.94** | 78.64 |
| Compound **8** | 400 | ig×14 | 8 | 8 | 4.22±0.75** | 64.09# |
| Compound **11** | 400 | ig×14 | 8 | 8 | 4.07±0.51 ** | 65.36# |
| Compound **13** | 400 | ig×14 | 8 | 8 | 3.83±0.64** | 67.40# |

Compared with the control group: *P<0.05, **P<0.01; compared with compound **6,** other compounds: #P<0.05 and ##P<0.01.

Based on the above experimental results: some compounds of the present invention had significant inhibitory effects on lung cancer, especially compound **6,** which had the best inhibitory effect on lung cancer and could be used to prepare medicaments for treating lung cancer.

**Experimental example 4: Efficacy of compounds on human liver cancer QGY7703 transplanted tumor in nude mice**

A human liver cancer QGY7703 model transplanted into nude mice was used in the experiment to investigate the anti-tumor effect of test compounds. The mice were intravenously injected at a dosage of 100 mg/kg for 14 days, and the tumor inhibition rate against human liver cancer QGY7703 was measured.

Preparation: The compound was prepared as the injection at the desired concentration with sterile normal saline, prior to administration.

Animal strain: BALB/C nude mice (SPF grade), weight: 19-21 g, gender: male.

Number of animals in each treatment group: 8; number of animals in the control group: >12.

**Experimental method:**

The tumor blocks of human liver cancer QGY7703 with good growth were cut into uniform small pieces of 3 mm under sterile conditions, and then subcutaneously inoculated into the right armpit of each mouse with a cannula. These mice were divided into 9 groups, 8 mice for each treatment group, while more than 12 mice were included in the control group.

14 days after inoculation, the average volume of the tumor block was about 100 $mm^3$. The animals were regrouped based on the size of the tumor, and the animals with tumors that were too large and too small were sifted out. For each group, the average volume of tumors was basically the same, and then the mice were initially treated with test compounds. The mice in the treatment groups were intravenously injected once a day for 14 days, at a dosage of 0.1 mL/20 g body weight; the mice in the control group were given the same volume of sterile normal saline. From the 14th day of inoculation, the long diameter a (mm) of the tumor, together with the perpendicular short diameter b (mm), was measured using a digital electronic caliper, twice a week. The calculation formula of tumor volume is: $TV = ab^2/2$, and the formula for calculating the relative tumor volume is RTV = Vt/Vo, wherein Vo denotes the tumor volume measured when the mice were divided into groups (i.e. d1), and Vt is the tumor volume from each measurement.

The result determination is based on the following formula:

$$\text{Tumor inhibitory rate\%} = \frac{\text{The average RTV of the control group - The average RTV of the treatment group}}{\text{The average RTV of the control group}} \times 100\%$$

The experimental results are shown in Table 5:

Table 5. The inhibitory effect of compounds on the tumor mass of liver cancer QGY7703.

| Group | Dosage (mg/kg) | Dosage regimen | Animal number | | RTV (d18) | Tumor inhibitory rate % |
|---|---|---|---|---|---|---|
| | | | Beginning | End | | |
| Control | | | 16 | 16 | 12.86±1.23 | |
| Compound **2** | 100 | iv×14 | 8 | 8 | 4.51±0.58** | 64.93# |
| Compound **3** | 100 | iv×14 | 8 | 8 | 4.67±0.63** | 63.69# |
| Compound **4** | 100 | iv×14 | 8 | 8 | 5.11±0.76** | 60.26## |
| Compound **6** | 100 | iv×14 | 8 | 8 | 3.65±0.47** | 71.62 |
| Compound **7** | 100 | iv×14 | 8 | 8 | 4.63±0.75** | 64.00# |
| Compound **9** | 100 | iv×14 | 8 | 8 | 4.67±0.58** | 63.69# |
| Compound **10** | 100 | iv×14 | 8 | 8 | 4.98±0.72** | 61.27## |
| Compound **12** | 100 | iv×14 | 8 | 8 | 4.86±0.59** | 62.20# |

Compared with the control group: *P<0.05, **P<0.01; compared with compound **6,** other compounds: #P<0.05 and ##P<0.01.

Based on the above experimental results: some compounds of the present invention had significant inhibitory effects on liver cancer, especially compound **6,** which had the best inhibitory effect on liver cancer and could be used to prepare medicaments for treating liver cancer.

**Experimental example 5: The efficacy of compounds on human gastric cancer MGC803 transplanted tumor in nude mice**

A human gastric cancer MGC803 model transplanted into nude mice was used in the experiment to investigate the anti-tumor effect of test compounds. The mice were intraperitoneally injected at a dosage of 200 mg/kg for consecutive 14 days, and the tumor inhibition rate against human gastric cancer MGC803 was measured.
Preparation: The compound was prepared as the injection at the desired concentration with sterile normal saline, prior to administration.
Animal strain: BALB/C nude mice (SPF grade), weight: 19-21 g, gender: male.
Number of animals in each treatment group: 8; number of animals in the control group: >12.

**Experimental method:**

The tumor blocks of human gastric cancer MGC803 with good growth were cut into uniform small pieces of 3 mm under sterile conditions, and then subcutaneously inoculated into the right armpit of each mouse with a cannula. These mice were divided into 9 groups, 8 mice for each treatment group, while more than 12 mice were included in the control group.
14 days after inoculation, the average volume of the tumor block was about 100 $mm^3$. The animals were regrouped based on the size of the tumor, and the animals with tumors that were too large and too small were sifted out. For each group, the average volume of tumors was basically the same, and then the mice were initially treated with test compounds. The mice were intraperitoneally injected once a day for 14 days, at a dosage of 0.2 mL/20 g body weight. From the 14th day of inoculation, the long diameter a (mm) of the tumor, together with the perpendicular short diameter b (mm), was measured using a digital electronic caliper, twice a week. The calculation formula of tumor volume is: $TV = ab^2/2$, and the formula for calculating the relative tumor volume is RTV = Vt/Vo, wherein Vo denotes the tumor volume measured when the mice were divided into groups (i.e. d1), and Vt is the tumor volume from each measurement.
The result determination is based on the following formula:

$$\text{Tumor inhibitory rate\%} = \frac{\text{The average RTV of the control group - The average RTV of the treatment group}}{\text{The average RTV of the control group}} \times 100\%$$

The experimental results are shown in Table 6:

Table 6. The inhibitory effect of compounds on the tumor mass of gastric cancer MGC803.

| Group | Dosage (mg/kg) | Dosage regimen | Animal number | | RTV (d18) | Tumor inhibitory rate % |
|---|---|---|---|---|---|---|
| | | | Beginning | End | | |
| Control | | | 16 | 16 | 11.52±1.23 | |
| Compound 1 | 200 | ip×14 | 8 | 8 | 5.87±0.63** | 49.05## |
| Compound **3** | 200 | ip×14 | 8 | 8 | 4.79±0.5 4** | 58.42## |
| Compound **6** | 200 | ip×14 | 8 | 8 | 3.43±0.51** | 70.23 |
| Compound **7** | 200 | ip×14 | 8 | 8 | 4.46±0.62** | 61.28## |
| Compound **8** | 200 | ip×14 | 8 | 8 | 4.59±0.47** | 60.16## |
| Compound **10** | 200 | ip×14 | 8 | 8 | 4.21±0.58** | 63.45# |
| Compound **11** | 200 | ip×14 | 8 | 8 | 4.72±0.61** | 59.03## |

Compared with the control group: *P<0.05, **P<0.01; compared with compound **6,** other compounds: #P<0.05 and ##P<0.01.
Based on the above experimental results: some compounds of the present invention had significant inhibitory effects on gastric cancer, and especially, compound **6** had the best inhibitory effect on gastric cancer and could be used in the preparation of medicaments for treating gastric cancer.

**Experimental example 6: The efficacy of compounds on human ovarian cancer A2780 transplanted tumor in nude mice**

A human ovarian cancer A2780 model transplanted into nude mice was used in the experiment to investigate the anti-tumor effect of test compounds. The mice were intraperitoneally injected at a dosage of 200 mg/kg for successive 14 days, and the tumor inhibition rate against human ovarian cancer A2780 was measured.
Preparation: The compound was prepared as the injection at the desired concentration with sterile normal saline, prior to administration.

Animal strain: BALB/C nude mice (SPF grade), weight: 19-21 g, gender: male.

Number of animals in each treatment group: 8; number of animals in the control group: >12.

**Experimental method:**

The tumor blocks of human ovarian cancer A2780 growing well were cut into uniform small pieces of 3 mm under sterile conditions, and then subcutaneously inoculated into the right armpit of each mouse with a cannula. The mice were divided into 9 groups, 8 mice for each treatment group, while >12 mice were included in the control group.
14 days after inoculation, the average volume of the tumor block was about 100 $mm^3$. The animals were regrouped based on the size of the tumor, and the animals with tumors that were too large and too small were sifted out. For each group, the average volume of tumors was basically the same, and then the mice were initially treated with test compounds. The mice were intraperitoneally injected once a day for 14 days, at a dosage of 0.2 mL/20 g body weight; the mice in the control group were given the same volume of sterile normal saline. From the 14th day of inoculation, the long diameter a (mm) of the tumor, together with the perpendicular short diameter b (mm), was measured using a digital electronic caliper, twice a week. The calculation formula of tumor volume is: $TV = ab^2/2$, and the formula for calculating the relative tumor volume is RTV = Vt/Vo, wherein Vo denotes the tumor volume measured when the mice were divided into groups (i.e. d1), and Vt is the tumor volume from each measurement.
The result determination is based on the following formula:

$$\text{Tumor inhibitory rate\%} = \frac{\text{The average RTV of the control group - The average RTV of the treatment group}}{\text{The average RTV of the control group}} \times 100\%$$

The experimental results are shown in Table 7:

Table 7. The inhibitory effect of compounds on the tumor mass of ovarian cancer A2780.

| Groups | Dosage (mg/kg) | Dosage regimen | Animal number Beginning | Animal number end | RTV (d18) | Tumor inhibitory rate % |
|---|---|---|---|---|---|---|
| Control | | | 16 | 16 | 10.31±0.87 | |
| Compound **1** | 200 | ip×14 | 8 | 8 | 5.63±0.72* | 45.39## |
| Compound **4** | 200 | ip×14 | 8 | 8 | 4.51±0.67** | 56.26## |
| Compound **6** | 200 | ip×14 | 8 | 8 | 3.58±0.49** | 65.28 |
| Compound **8** | 200 | ip×14 | 8 | 8 | 5.21±0.47** | 49.47## |
| Compound **9** | 200 | ip×14 | 8 | 8 | 4.37±0.62** | 57.61# |
| Compound **10** | 200 | ip×14 | 8 | 8 | 4.26±0.73** | 58.68# |
| Compound **13** | 200 | ip×14 | 8 | 8 | 4.78±0.83** | 53.64## |

Compared with the control group: *P<0.05, **P<0.01; compared with compound **6,** other compounds: #P<0.05 and ##P<0.01.
Based on the above experimental results: some compounds of the present invention had significant inhibitory effects on ovarian cancer, and especially, compound 6 had the best inhibitory effect on ovarian cancer and could be used in the preparation of medicaments for treating ovarian cancer.

**Experimental example 7: Efficacy of compounds on human prostate cell LNCaP transplanted tumor in nude mice**

A human prostate cell LNCaP model transplanted into nude mice was used in the experiment to investigate the anti-tumor effect of test compounds. The mice were intraperitoneally injected at a dosage of 200 mg/kg for successive 14 days, and the tumor inhibition rate against human prostate cell LNCaP was measured.

Preparation: The compound was prepared as the injection at the desired concentration with sterile normal saline, prior to administration.

Animal strain: BALB/C nude mice (SPF grade), weight: 19-21 g, gender: male.

Number of animals in each treatment group: 8; number of animals in the control group: >12.

**Experimental method:**

The tumor blocks of human prostate cell LNCaP with good growth were cut into uniform small pieces of 3 mm under sterile conditions, and then subcutaneously inoculated into the right armpit of each mouse with a cannula. The mice were divided into 9 groups, 8 mice for each treatment group, while >12 mice were included in the control group.

14 days after inoculation, the average volume of the tumor block was about 100 $mm^3$. The animals were regrouped based on the size of the tumor, and the animals with tumors that were too large and too small were sifted out. For each group, the average volume of tumors was basically the same, and then the mice were initially treated with test compounds. The mice were intraperitoneally injected once a day for 14 days, at a dosage of 0.2 mL/20 g body weight; the mice in the control group were given the same volume of sterile normal saline. From the 14th day of inoculation, the long diameter a (mm) of the tumor, together with the perpendicular short diameter b (mm), was measured using a digital electronic caliper, twice a week. The calculation formula of tumor volume is: TV = $ab^2/2$, and the formula for calculating the relative tumor volume is: RTV = Vt/Vo, wherein Vo denotes the tumor volume measured when the mice were divided into groups (i.e. d1), and Vt denotes the tumor volume from each measurement.

The result determination is based on the following formula:

$$\text{Tumor inhibitory rate\%} = \frac{\text{The average RTV of the control group - The average RTV of the treatment group}}{\text{The average RTV of the control group}} \times 100\%$$

The experimental results are shown in Table 8:

Table 8. The inhibitory effect of compounds on the tumor mass of prostate cell LNCaP.

| Group | Dosage (mg/kg) | Dosage regimen | Animal number Beginning | Animal number end | RTV (d18) | Tumor inhibitory rate % |
|---|---|---|---|---|---|---|
| Control | | | 16 | 16 | 12.19±0.95 | |
| Compound **1** | 200 | ip×14 | 8 | 8 | 4.92±0.82* | 56.27## |
| Compound **3** | 200 | ip×14 | 8 | 8 | 4.77±0.71** | 60.87## |
| Compound **4** | 200 | ip×14 | 8 | 8 | 3.73±0.60** | 69.40 |
| Compound **6** | 200 | ip×14 | 8 | 8 | 4.27±0.92* | 64.97# |
| Compound **8** | 200 | ip×14 | 8 | 8 | 6.72±0.85* | 44.87## |
| Compound **10** | 200 | ip×14 | 8 | 8 | 4.55±0.81** | 62.67# |

Compared with the control group: *P<0.05, **P<0.01; compared with compound **4,** other compounds: #P<0.05 and ##P<0.01.

Based on the above experimental results: some compounds of the present invention had significant inhibitory effects on prostate cancer, and especially, compound **4** had the best inhibitory effect on prostate cancer and could be used in the preparation of medicaments for treating prostate cancer.

**Experimental example 8: Efficacy of compounds on human renal cell carcinoma AGHN transplanted tumor in nude mice**

A human renal cell carcinoma AGHN model transplanted into nude mice was used in the experiment to investigate the anti-tumor effect of test compounds. The mice were intraperitoneally injected at a dosage of 200 mg/kg for successive 14 days, and the tumor inhibition rate against human renal cell carcinoma AGHN was measured.
Preparation: The compound was prepared as the injection at the desired concentration with sterile normal saline, prior to administration.
Animal strain: BALB/C nude mice (SPF grade), weight: 19-21 g, gender: male.
Number of animals in each treatment group: 8; number of animals in the control group: >12.

**Experimental method:**

The tumor blocks of human renal cell carcinoma AGHN with good growth were cut into uniform small pieces of 3 mm under sterile conditions, and then subcutaneously inoculated into the right armpit of each mouse with a cannula. The mice were divided into 9 groups, 8 mice for each treatment group, while >12 mice were included in the control group.
14 days after inoculation, the average volume of the tumor block was about 100 $mm^3$. The animals were regrouped based on the size of the tumor, and the animals with tumors that were too large and too small were sifted out. For each group, the average volume of tumors was basically the same, and then the mice were initially treated with test compounds. The mice were intraperitoneally injected once a day for 14 days, at a dosage of 0.2 mL/20 g body weight; the mice in the control group were given the same volume of sterile normal saline. From the 14th day of inoculation, the long diameter a (mm) of the tumor, together with the perpendicular short diameter b (mm), was measured using a digital electronic caliper, twice a week. The calculation formula of tumor volume is: TV = $ab^2/2$, and the formula for calculating the relative tumor volume is: RTV = Vt/Vo, wherein Vo denotes the tumor volume measured when the mice were divided into groups (i.e. d1), and Vt denotes the tumor volume from each measurement.
The result determination is based on the following formula:

$$\text{Tumor inhibitory rate\%} = \frac{\text{The average RTV of the control group - The average RTV of the treatment group}}{\text{The average RTV of the control group}} \times 100\%$$

The experimental results are shown in Table 9:

Table 9. The inhibitory effect of compounds on the tumor mass of renal cell carcinoma AGHN.

| | | | Animal number | | | |
|---|---|---|---|---|---|---|
| Group | Dosage (mg/kg) | Dosage regimen | Beginning | End | RTV (d18) | Tumor inhibitory rate % |
| Control | | | 16 | 16 | 13.79±0.82 | |
| Compound **1** | 200 | ip×14 | 8 | 8 | 6.31±0.91* | 54.24## |
| Compound **3** | 200 | ip×14 | 8 | 8 | 5.47±0.82** | 60.33# |
| Compound **4** | 200 | ip×14 | 8 | 8 | 4.31±0.58** | 68.75 |
| Compound **6** | 200 | ip×14 | 8 | 8 | 6.53±0.85** | 52.64## |
| Compound **8** | 200 | ip×14 | 8 | 8 | 6.22±0.63* | 54.89## |
| Compound **9** | 200 | ip×14 | 8 | 8 | 5.12±0.77** | 62.87# |
| Compound **12** | 200 | ip×14 | 8 | 8 | 5.62±0.59** | 58.48## |

Compared with the control group: *P<0.05, **P<0.01; compared with compound **4,** other compounds: #P<0.05, ##P<0.01.
Based on the above experimental results: some compounds of the present invention had significant inhibitory effects on renal cell carcinoma, and especially, compound **4** had the best inhibitory effect on renal cell carcinoma and could be used in the preparation of medicaments for treating renal cell carcinoma.

**Experimental example 9: Compound 6 causing tumor necrosis *in vivo***

**1. Experimental methods**

Well growing solid tumors (human lung cancer 95D and human liver cancer Bel7404) were cut into uniform small pieces of approximate 2-3 mm under sterile conditions, and subcutaneously inoculated into the right armpit of each nude mouse using a cannula. 14 days after inoculation, each mouse was treated with compound **6** by oral administration at a dosage of 400 mg/kg (in a dosage by volume of 0.5 mL/20 g body weight), and compound **6** was prepared as a suitable solution with sterile normal saline. At different time points (4 h, 24 h) after administration, the nude mice were killed, and the tumor tissue was dissected and fixed with 10% formalin. The pathological sections were made, followed by HE staining and taking photos, to observe the internal necrosis and cell proliferation within the tumors.

**2. Experimental results:**

HE staining is shown in Figures 2 and 3. 4 h after oral administration of compound **6** (400 mg/kg), necrosis began to appear within the tumor tissues; 24 h after administration, compound **6** can cause massive necrosis (light colored areas) within the tumor tissue. This indicated that compound **6** of the present invention could effectively cause tumor necrosis and achieve anti-tumor effects.

**Experimental example 10: Compound 6 causing *in vivo* vascular necrosis of tumors**

**1. Experimental methods**

Well growing solid tumors (human lung cancer 95D and human liver cancer Bel7404) were cut into uniform small pieces of approximate 2-3 mm under sterile conditions, and subcutaneously inoculated into the right armpit of each nude mouse using a cannula. 14 days after inoculation, each mouse was treated with compound **6** by oral administration at a dosage of 400 mg/kg (a dosage by volume of 0.5 mL/20 g body weight), and compound **6** was prepared as a suitable solution with sterile normal saline. At different time points (4 h, 24 h) after administration, the nude mice were killed, and the tumor tissue was dissected and fixed with 10% formalin. The pathological slices were made, followed by HE staining and photographing, to observe the damage to the blood vessels of tumors.

**2. Experimental results:**

PCNA immunohistochemistry showed (Figures 4 and 5): 4 h after oral administration of compound **6** (400 mg/kg), only some vascular endothelial cells proliferated in the tumor tissues. While, 24 h after administration, compound **6** could cause massive blood flow blockade and necrosis of tumor tissues. The downregulation of PCNA expression in tumor tissues indicated that compound **6** had better *in vivo* destructive effects on tumor blood vessels.

**Experimental example 11: The inhibitory effects of compound 3 combined with lenvatinib on the growth of human liver cancer Bel7404 xenograft in nude mice**

Experimental method: The model of human liver cancer Bel7404 transplant tumor in nude mice was established by subcutaneously inoculating human liver cancer Bel7404 cell lines into the armpits of nude mice. Cells were inoculated in an amount of $1 \times 10^6$, and after formation of the transplanted tumors by inoculation, the cells were passed 3 generations in nude mice before use. The vigorous growth of tumor tissue was cut into approximate 1.5 $mm^3$, and homogenized under sterile conditions, to prepare $1\times10^7$/mL of cell suspension. 0.1 mL of cell suspension was subcutaneously inoculated into the right armpit of nude mice. For transplanted tumors in nude mice, the diameter of the transplanted tumor was measured with vernier caliper. After the tumor grew to 100 $mm^3$, the animals were randomly divided into groups, with 6 animals in each group. Using the method of measuring tumor diameter, the anti-tumor effect of test compound was dynamically observed. Compound **3** was orally administered at 400 mg/kg, once a day, for three weeks; lenvartinib was also orally administered at 10 mg/kg, once a day, for two weeks. The measurement frequency of tumor diameter was once every three days. The animals in the negative control group were orally given an equal amount of physiological saline. The effect of combined medicaments was evaluated based on the Q value calculated by the Jin's formula:

$$Q = Ea{+}b \,/\, (Ea + Eb - Ea \times Eb)$$

Ea+b represents the tumor inhibitory rate of the combined medicaments, while Ea and Eb represent the tumor

inhibitory rates of medicament A and medicament B, respectively. For example, the Q value of 0.85-1.15 means additive action (+), and the Q value of >1.15 means synergistic action (++).

The experimental results are shown in Table 10:

Table 10. The inhibitory effects of compound **3** combined with lenvatinib on the growth of human liver cancer Bel7404 xenograft in nude mice.

| Group | Dosage (mg/kg.po) | Animal number Beginni ng/End | Terminal weight (g) | RTV (d21) | Tumor inhibitory rate (%) | Q value |
|---|---|---|---|---|---|---|
| Control | - | 6/6 | 22.7±0.6 | 11.23±1.75 | - | |
| Lenvatinib | 10 | 6/6 | 21.8±0.7 | 3.89±1.46* | 65.36 | |
| Compound **3** | 400 | 6/6 | 22.4±0.6 | 3.54±1.87* | 68.47 | |
| Lenvatinib + compound **3** | 10+400 | 6/6 | 23.3±0.5 | 1.53+0.89** | 86.38 | 0.970 |

Compared with the blank control group, *$P < 0.05$

**Experimental results:**

Compound **3** combined with lenvatinib had a Q value of 0.970, indicating a significant anti-tumor additive effect, but no synergistic anti-tumor effect. No obvious decrease in animal weight was observed, and the mice did not show obvious toxic addition reaction after treatment with compound **3** in combination with lenvatinib.

**Experimental example 12: The inhibitory effects of compound 6 combined with paclitaxel on the growth of human gastric cancer SGC7901 xenograft in nude mice**

Experimental method: The model of human gastric cancer SGC7901 transplant tumor in nude mice was established by subcutaneously inoculating human gastric cancer SGC7901 cell lines into the armpits of nude mice. Cells were inoculated in an amount of $1 \times 10^6$, and after formation of the transplanted tumors by inoculation, the cells were passed 3 generations in nude mice before use. The vigorous growth of tumor tissue was cut into approximate 1.5 $mm^3$, and homogenized under sterile conditions, to prepare $1\times10^7$/mL of cell suspension. 0.1 mL of cell suspension was subcutaneously inoculated into the right armpit of nude mice. For transplanted tumors in nude mice, the diameter was measured with vernier caliper. After the tumor grew to 100 $mm^3$, the animals were randomly divided into groups, with 6 mice in each group. Using the method of measuring tumor diameter, the anti-tumor effect of test compound was dynamically observed. Compound **6** was administered at 200 mg/kg by intraperitoneal injection, once every other day, for three weeks; paclitaxel was administered at 10 mg/kg by intravenous injection, once every other day, for three weeks. The measurement frequency of tumor diameter was once every three days. The dosage by volume was 0.1 mL/20 g. The mice in the negative control group were given an equal amount of physiological saline by intravenous injection. The effect of combined medicaments was evaluated based on the Q value calculated by Jin's formula:

$$Q = Ea+b / (Ea+Eb-Ea\times Eb)$$

Ea+b represents the tumor inhibitory rate of the combined medicaments, while Ea and Eb represent the tumor inhibitory rates of medicament A and medicament B, respectively. For example, the Q value of 0.85-1.15 means additive action (+), while the Q value of >1.15 means synergistic action (++).

The experimental results are shown in Table 11:

Table 11. The inhibitory effects of compound 6 combined with paclitaxel on the growth of human gastric cancer SGC7901 xenograft in nude mice.

| Group | Dosage (mg/kg) | Animal number Beginning /End | Terminal weight (g) | RTV (d21) | Tumor inhibitory rate (%) | Q value |
|---|---|---|---|---|---|---|
| Control | - | 6/6 | 22.8±0.9 | 13.52±2.41 | - | |

(continued)

| Group | Dosage (mg/kg) | Animal number Beginning /End | Terminal weight (g) | RTV (d21) | Tumor inhibitory rate (%) | Q value |
|---|---|---|---|---|---|---|
| Paclitaxel | 10×iv | 6/6 | 21.7±0.6* | 7.37±1.76* | 45.49 | |
| Compound **6** | 200×ip | 6/6 | 22.9+1.2* | 6.21±1.88* | 54.07 | |
| Paclitaxel + compound **6** | 10+200 | 6/6 | 21.9±0.8 | 4.13±1.41* | 69.45 | 0.927 |

Compared with the blank control group, *P < 0.05

**Experimental results:**

The Q value for compound **6** in combination with paclitaxel was 0.927, indicating a significant anti-tumor additive effect, without synergistic anti-tumor effect. There was no obvious decrease in animal weight, and when combined with paclitaxel, the mice in the treatment group of paclitaxel + compound **6** did not show obvious toxic addition reaction.

**Experimental example 13: The inhibitory effects of compound 8 combined with carboplatin on the growth of human lung cancer A549 xenograft in nude mice**

Experimental method: The model of human lung cancer A549 transplant tumor in nude mice was established by subcutaneously inoculating human lung cancer A549 cell lines into the armpits of nude mice. Cells were inoculated in an amount of $1 \times 10^6$, and after formation of the transplanted tumors by inoculation, the cells were passed 3 generations in nude mice before use. The vigorous growth of tumor tissue was cut into approximate 1.5 $mm^3$, and homogenized under sterile conditions, to prepare $1\times10^7$/mL of cell suspension. 0.1 mL of cell suspension was subcutaneously inoculated into the right armpit of nude mice. For transplanted tumors in nude mice, the diameter was measured with vernier caliper. After the tumor grew to 100 $mm^3$, the animals were randomly divided into groups, with 6 mice in each group. Using the method of measuring tumor diameter, the anti-tumor effect of test compound was dynamically observed. Compound **8** was administered at 200 mg/kg by intraperitoneal injection, once every other day, for three weeks; carboplatin was administered at 10 mg/kg by intraperitoneal injection, once every other day, for three weeks. The measurement frequency of tumor diameter was once every three days. The dosage by volume was 0.1 mL/20 g. The mice in the negative control group were given an equal amount of physiological saline by intravenous injection. The effect of combined medicaments was evaluated based on the Q value calculated by Jin's formula:

$$Q = Ea+b / (Ea+Eb-Ea\times Eb)$$

Ea+b represents the tumor inhibitory rate of the combined medicaments, while Ea and Eb represent the tumor inhibitory rates of medicament A and medicament B, respectively. For example, the Q value of 0.85-1.15 means additive action (+), while the Q value of >1.15 means synergistic action (++).

The experimental results are shown in Table 12:

Table 12. The inhibitory effects of compound **8** combined with carboplatin on the growth of human liver cancer A549 xenograft in nude mice.

| Group | Dosage (mg/kg.ip) | Animal number Beginni ng/End | Terminal weight (g) | RTV(d21) | Tumor inhibitory rate (%) | Q value |
|---|---|---|---|---|---|---|
| Control | - | 6/6 | 23.1+1.3 | 12.87±1.93 | - | |
| Carboplatin | 10 | 6/6 | 21.5±0.9* | 6.63±1.42* | 48.48 | |
| Compound 8 | 200 | 6/6 | 23.4+1.3* | 4.85±1.37* | 62.32 | |
| Carboplatin + compound 8 | 10+200 | 6/6 | 21.7±0.8 | 3.11±0.93* | 75.83 | 0.941 |

Compared with the blank control group, *P < 0.05

**Experimental results:**

The Q value for compound **8** combined with carboplatin was 0.941, indicating a significant anti-tumor additive effect, without synergistic anti-tumor effect. There was no obvious decrease in animal weight, but when combined with carboplatin, the mice in the treatment group of carboplatin + compound **8** did not show obvious toxic addition reaction.

**Experimental example 14: The tumor inhibitory effect of compound 11 combined with PD-L1 on Lewis non-small cell lung cancer model in mice**

Experimental method: The model of transplant tumor in C57BL/6 mice was established by subcutaneously inoculating Lewis non-small cell lung cancer cell lines into the armpits of C57BL/6 mice. Cells were inoculated in an amount of $1 \times 10^6$, and after formation of the transplanted tumors by inoculation, the cells were passed 3 generations in C57BL/6 mice before use. The vigorous growth of tumor tissue was cut into approximate 1.5 $mm^3$, and homogenized under sterile conditions, to prepare $1\times10^7$/mL of cell suspension. 0.1 mL of cell suspension was subcutaneously inoculated into the right armpit of C57BL/6 mice. For transplanted tumors in C57BL/6 mice, the diameter was measured with vernier caliper. After the tumor grew to 100 $mm^3$, the animals were randomly divided into groups, with 6 mice in each group. Using the method of measuring tumor diameter, the anti-tumor effect of test compound was dynamically observed. Compound **11** was administered at 200 mg/kg by intraperitoneal injection, once every other day, for three weeks; PD-L1 was administered at 10 mg/kg by intraperitoneal injection, once every other day, for three weeks. The measurement frequency of tumor diameter was once every three days. The dosage by volume was 0.1 mL/20 g. The mice in the negative control group were given an equal amount of physiological saline by intravenous injection.

The effect of combined medicaments was evaluated based on the Q value calculated by Jin's formula:

$$Q = Ea{+}b / (Ea{+}Eb{-}Ea{\times}Eb)$$

Ea+b represents the tumor inhibitory rate of the combined medicaments, while Ea and Eb represent the tumor inhibitory rates of medicament A and medicament B, respectively. For example, the Q value of 0.85-1.15 means additive action (+), while the Q value of >1.15 means synergistic action (++).

The experimental results are shown in Table 13:

Table 13. The tumor inhibitory effect of compound **11** combined with PD-L1 on Lewis non-small cell lung cancer model in mice.

| Group | Dosage (mg/kg.ip) | Animal number Beginning /End | Terminal weight (g) | RTV (d18) | Tumor inhibitory rate (%) | Q value |
|---|---|---|---|---|---|---|
| Control | - | 6/6 | 22.5±1.1 | 14.87±2.76 | - | |
| PD-L1 | 10 | 6/6 | 22.8±0.7 | 8.56±2.33* | 42.43 | |
| Compound **11** | 200 | 6/6 | 23.1±0.9 | 7.62±2.28* | 48.76 | |
| PD-L1 + compound **11** | 10+200 | 6/6 | 22.7+0.9 | 2.73±0.92* | 81.64 | 1.16 |

Compared with the blank control group, *P < 0.05

**Experimental results:**

The Q value for compound **11** combined with PD-L1 was 1.16, indicating the combination of compound **11** and PD-L1 had a significant synergistic anti-tumor effect. There was no obvious decrease in animal weight, and the mice in the treatment group of PD-L1 + compound **11** did not show obvious toxic addition reaction.

**Experimental example 15: The tumor inhibitory effect of compound 6 combined with T cells on human liver cancer QGY7703 model in NOG mice**

Experimental method: QGY7703 tumor cells were washed twice with PBS, and then resuspended with PBS:Matrix (1:1 mixture by volume). The cell concentration was adjusted to 2.5 × $10^6$ /mL, and subcutaneously inoculated to the right armpit of experimental animals at 200 μL/mouse, i.e. 5 × $10^5$ cells/mouse. When the tumor grew to about 50 $mm^3$, the mice were divided into groups for administration.

T cell culture and harvest: PBMCs of donors were activated and expanded *in vitro* according to the standard operating procedures for T cell culture. The cells in logarithmic growth phase were harvested and suspended in the medium at a moderate dose 2×10'/mL) for future use.

When the tumor grew to the suitable size, separate injection of T cell and compound **6** was performed, and the time was recorded as D0. Compound **6** was intraperitoneally injected at a dose of 200 mg/kg. Starting from day 0 (D0), compound **6** was administered daily for successive 14 days. T cells were administrated by subcutaneous injection, and the injection site was the same as the inoculation site of tumor cells. The administration of T cells was carried out on D0 by three injections, and the injection regimen is shown in Table 14 below. T cells were administered approximate 4 hours after administration of compound **6.**

The general conditions, body weight, tumor volume, and tumor weight of mice were recorded every 3 days. Based on the status of the tumor, whether EAL was further injected was decided.

Table 14. Dosage regimen.

| Group | Injection route | EAL concentration (cells/ml) | Injection volume (mL) | EAL amount (cells/ mouse) |
|---|---|---|---|---|
| D0 administration | Subcutaneous injection | 2×$10^7$ | 0.1 | 2×$10^6$ |
| D1 | Subcutaneous | 2×$10^7$ | 0.2 | 4×$10^6$ |
| administration | injection | | | |
| D2 administration | Subcutaneous injection | 2×$10^7$ | 0.2 | 4×$10^6$ |

The effect of combined medicaments was evaluated based on the Q value calculated by Jin's formula:

$$Q = Ea+b / (Ea+Eb-Ea\times Eb)$$

Ea+b represents the tumor inhibitory rate of the combined medicaments, while Ea and Eb represent the tumor inhibitory rates of medicament A and medicament B, respectively. For example, the Q value of 0.85-1.15 means additive action (+), while the Q value of >1.15 means synergistic action (++).

The experimental results are shown in Table 15:

Table 15. The tumor inhibitory effect of compound 6 combined with T cells on human liver cancer QGY7703 model in NOG mice.

| Group | Dosage (mg/kg.ip) | Animal number Beginning/ End | Terminal weight (g) | RTV(d32) | Tumor inhibitory rate (%) | Q value |
|---|---|---|---|---|---|---|
| Control | - | 6/6 | 24.8±1.2 | 32.49±5.22 | - | |
| T cells | 8×$10^6$ T cells | 6/6 | 23.9±1.3 | 25.37±4.763 | 21.91 | |
| Compound 6 | 200.ip | 6/6 | 24.5±0.9 | 19.62±3.17* | 39.61 | |
| T cells + Compound 6 | 8×$10^6$ T cells + 200.ip | 6/6 | 23.7±1.4 | 11.91±2.82* | 63.34 | 1.20 |

Compared with the blank control group, *P < 0.05

**Experimental results:**

The Q value for compound **6** combined with T cells was 1.20, indicating the combination of compound **6** and T cells had a significant synergistic effects on liver cancers. There was no obvious decrease in animal weight, and the mice in the treatment group of T cells + compound **6** did not show obvious toxic addition reaction.

Experimental examples 14 and 15 demonstrated that the stilbene compounds of the present invention could be used in combination with tumor immunotherapy drugs to play synergistic anti-tumor effects.

**Experimental example 16: The inhibitory effect of compound 6 on tumor metastasis**

Under sterile conditions, B16 mouse melanoma cells in logarithmic growth period were cultured, and then prepared as $2.8\times10^5$/mL of cell suspension, which was inoculated into the tail vein of C57BL/6 mice at 0.2 mL/mouse. Compound 6 was administered to the mice on the next day. The dosage regimen was shown in Table 16. Three weeks later, each animal was sacrificed, and the lungs of mice in each group were dissected. The number of metastatic colonies in the lungs of mice was detected and counted, and the average number of tumor colonies in each group was used to calculate the tumor inhibitory rate according to the formula: Tumor inhibitory rate % = [(The average number of colonies in the control group - the average number of colonies in the treatment group) / The average number of colonies in the control group] $\times$ 100%. The results are shown in Table 16.

Table 16. Anti-metastatic action of compound **6** on lung metastasis model of B16 mouse melanoma.

| Sample | Dosage mg/kg | Dosage regimen | Animal number Beginning/End | Animal weight (g) Beginning/End | Number of lung tumor colonies $\overline{X}\pm$SD | Metastasis inhibitory rate % |
|---|---|---|---|---|---|---|
| Negative control | Normal saline | iv×10qod | 20/19 | 19.9/25.8 | 52.8±17.3 | |
| Compound 6 | 120 | iv×10qod | 10/10 | 20.2/25.6 | 34.8±13.4** | 34.14 |
| Compound 6 | 80 | iv×10qod | 10/10 | 19.9/25.7 | 33.9±10.8** | 35.84 |
| Compound 6 | 60 | iv×10qod | 10/10 | 20.1/25.7 | 37.5±11.7 | 29.03 |
| Compound 6 | 40 | iv×10qod | 10/10 | 20.2/25.4 | 40.1±9.0 | 24.11 |

**P<0.01 vs negative control.

The results showed that compound **6** could inhibit the metastasis of B 16 mouse melanoma in a dose-dependent manner, which might be related to inhibiting the movement of tumor thrombus.

**Experimental example 17: Investigating the inhibition of compound 6 on neovascularization of ocular surface in rats**

Four SD rats, regardless of gender, were subjected to single eye modeling, with a total of four eye models. One eye was used as a blank control (untreated group), one eye was used as a model control, one eye was used as treatment group 1, and one eye was used as treatment group 2.

The rats were anesthetized with pentobarbital sodium (25-30 mg/kg, intraperitoneal injection), and two drops of oxybuprocaine hydrochloride eye drops were administrated to both eyes for ocular surface anesthesia. 30 μL of NaOH solution (1 mol/L) was placed with a pipette on a dry filter paper with a diameter of approximate 3 mm for 10 seconds. The moistened circular filter paper was gently applied to the center of the cornea (covering the corneoscleral margin) of the model eye, and a tight fit was kept for suitable time. The filter paper was removed, and then physiological saline was used to wash the eye, which was rinsed with a needle for 1 minute. After operation, ofloxacin eye ointment was applied to prevent infection (3 times/day, for 5 days).

From the beginning of modeling, on the first, third, fifth, seventh, and tenth days (D1, D3, D5, D7, and D10), the conjunctival congestion, corneal edema, and corneal neovascularization of the anterior eye segment were observed

with a slit-lamp.

On the 5th day (D5), neovascularization began to appear, and D5 was the first day of administrating the test compound (d1). Three eye drops were administered daily for 10 consecutive days (d10). On the 10th day after administration, the conjunctival congestion, corneal edema and corneal neovascularization of the anterior segment were observed with a slit-lamp. After completion of the experiment, the rats were euthanized.

Blank control group (untreated group): no any treatment, no modeling, no administration.

Model group: A circular filter paper (with a diameter of approximate 3 mm) moistened with 30 μL of NaOH (1 mol/L) was gently applied to the cornea for modeling, and anti infection treatment was performed after modeling; but no medicament was administrated.

Treatment group 1: After modeling, compound **6** was dissolved in physiological saline at a concentration of 10 mg/mL and administered at 50 μL/eye by dropping in the conjunctival sac of lower eyelid, 3 times/day, for 10 days;

Treatment group 2: After modeling, compound **6** was dissolved in physiological saline at a concentration of 25 mg/mL and administered at 50 μL/eye by dropping in the conjunctival sac of lower eyelid, 3 times/day, for 10 days.

The results are shown in Figure 6. For the treatment groups, the neovascularization of the ocular surface was reduced compared to the model group, and the vascularization area was significantly decreased in the high-dose group. Compound **6** had an inhibitory effect on the growth of ocular surface neovascularization, which presented a dose-effect relationship.

In summary, the present invention provided the use of stilbene compounds in the preparation of anti-tumor medicaments. In the present invention, it was found that stilbene compounds had a dual effect of enriching $CD8^+$ T lymphocytes in solid tumors and targeting to damage the inner wall of tumor blood vessels. The stilbenes could kill tumor cells by cytotoxic T lymphocyte antigen $CD8^+$, and block the blood supply to tumor tissues at the same time, and thus cause rapid necrosis in solid tumors, together with inhibition on tumor metastasis, thereby greatly improving the killing effects on tumors. The stilbene compounds of the present invention could be used in the preparation of anti-tumor medicaments, and also used in combination with tumor immunotherapeutics to achieve synergistic anti-tumor effects. Among these stilbene compounds, the compounds with different structures might have different anti-tumor effects and thus could be used in the preparation of different anti-tumor medicaments. In addition, the stilbene compounds of the present invention could inhibit the growth of ocular surface blood vessels and be used in the preparation of medicaments for treating various eye diseases. The stilbene compounds of the present invention had good application prospects.

## Claims

1. A compound represented by formula I, or salt thereof, or stereoisomer thereof, or solvate thereof for use in the treatment of tumor and/or for use in preventing and/or treating eye disease:

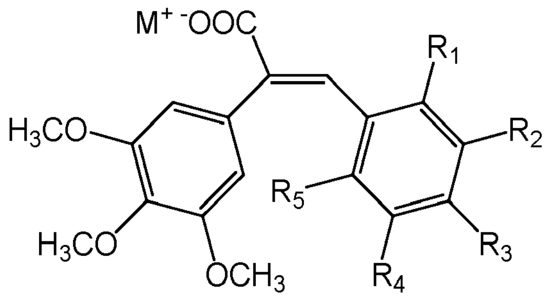

Formula I

wherein, M is selected from the group consisting of hydrogen, sodium, potassium, calcium, ammonium and organic amine;

$R_1$, $R_2$, $R_3$, $R_4$, and $R_5$ are each independently selected from the group consisting of hydrogen, $C_1$-$C_6$ alkyl, $C_1$-$C_6$ alkoxy, hydroxy, amino, and halogen.

2. The compound for use according to claim 1, **characterized in that** the organic amine is selected from the group consisting of amino acids, peptides, tert-butylamine and n-butylamine;

and/or, $R_1$, $R_2$, $R_3$, $R_4$, and $R_5$ are each independently selected from the group consisting of hydrogen, $C_1$-$C_3$ alkyl, methoxy, ethoxy, hydroxy, amino, fluorine, chlorine and bromine;

preferably,
the organic amine is selected from the group consisting of meglumine, pyrazine, alanine, n-butylamine, lysine and tert-butylamine;
and/or, $R_1$, $R_2$, $R_3$, $R_4$, and $R_5$ are each independently selected from the group consisting of hydrogen, methoxy, ethoxy, hydroxy, fluorine, chlorine and bromine.

3. The compound for use according to claim 1, **characterized in that** the compound is represented by formula II:

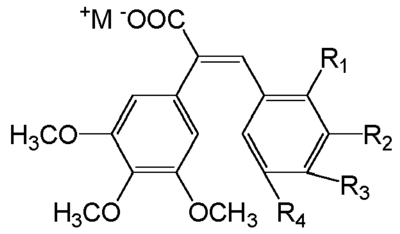

Formula II

wherein, M is selected from the group consisting of hydrogen, sodium, potassium, calcium, ammonium, meglumine, pyrazine, alanine, n-butylamine, lysine and tert-butylamine;
$R_1$, $R_2$, $R_3$, and $R_4$ are each independently selected from the group consisting of hydrogen, methoxy, ethoxy, hydroxy, fluorine, chlorine and bromine.

4. The compound for use according to claim 3, **characterized in that** the compound is represented by formula III:

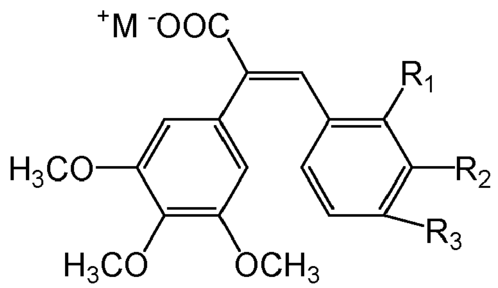

Formula III

wherein, M is selected from the group consisting of hydrogen, sodium, potassium, calcium, ammonium, meglumine, pyrazine, alanine, n-butylamine, lysine and tert-butylamine;
$R_1$, $R_2$, and $R_3$ are each independently selected from the group consisting of hydrogen, methoxy, ethoxy, hydroxy, fluorine, chlorine and bromine.

5. The compound for use according to claim 4, **characterized in that** the compound is represented by formula IV:

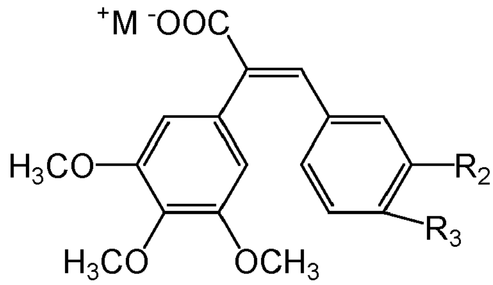

Formula IV

wherein, M is selected from the group consisting of hydrogen, sodium, potassium, calcium, ammonium, meglumine, pyrazine, alanine, n-butylamine, lysine and tert-butylamine;

$R_2$ and $R_3$ are each independently selected from the group consisting of hydrogen, methoxy, ethoxy, hydroxy, fluorine, chlorine and bromine.

6. The compound for use according to any one of claims 1 to 5, **characterized in that** it has the effect of enriching $CD8^+$ T lymphocytes in solid tumors and/or destroying the inner wall of tumor blood vessels;
preferably, cause the necrosis of tumor cells at the centers of solid tumors.

7. The compound for use according to any one of claims 1 to 5, **characterized in that** it inhibits tumor metastasis.

8. The compound for use according to claim 7, **characterized in that** when inhibiting tumor metastasis, the structure of the compound represented by formula I is as shown by formula Va:

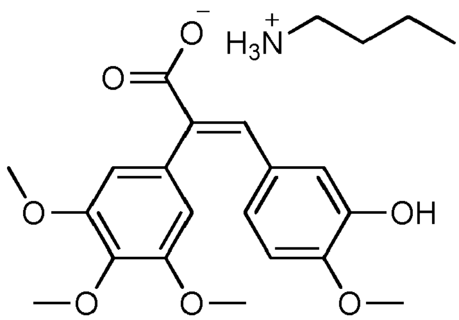

Formula Va;

preferably, inhibiting tumor metastasis is inhibiting melanoma metastasis.

9. The compound for use according to any one of claims 1 to 5, **characterized in that** tumor treatment comprises preventing and/or treating lung cancer, liver cancer, gastric cancer, ovarian cancer, prostate cancer, or renal cell carcinoma.

10. The compound for use according to claim 9, **characterized in that** for preventing and/or treating lung cancer, liver cancer, gastric cancer, or ovarian cancer, the structure of the compound represented by formula I is as shown by formula Va:

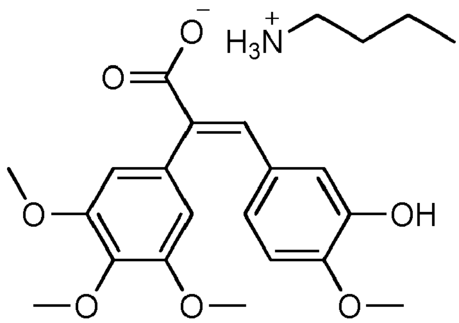

Formula Va.

**11.** The compound for use according to claim 9, **characterized in** for preventing and/or treating prostate cancer or renal cell carcinoma, the structure of the compound represented by formula I is as shown by formula Vb:

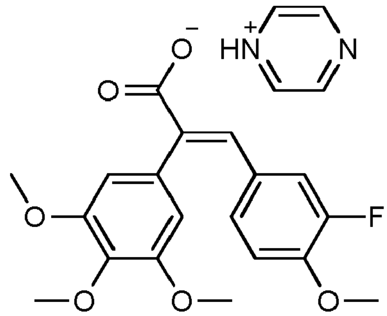

Formula Vb.

**12.** The compound for use according to any one of claims 1 to 5, **characterized in that** preventing and/or treating eye disease comprises inhibiting the growth of ocular surface blood vessels.

**13.** The compound for use according to any one of claims 1 to 5, **characterized in that** the eye disease is retinopathy, fundus hemangioma, fundus hemorrhage, dacryocystitis, glaucoma, cataract, vitreous opacity, optic atrophy, macular degeneration and/or retinal detachment.

**14.** The compound for use according to any one of claims 1 to 5, **characterized in that** the eye disease is diabetic ophthalmopathy,

preferably, the diabetic ophthalmopathy is retinopathy, fundus hemangioma, fundus hemorrhage, dacryocystitis, glaucoma, cataract, vitreous opacity, optic atrophy, macular degeneration and/or retinal detachment related to diabetes.

**15.** The compound for use according to any one of claims 12 to 14, **characterized in that** the structure of the compound is represented by formula Va:

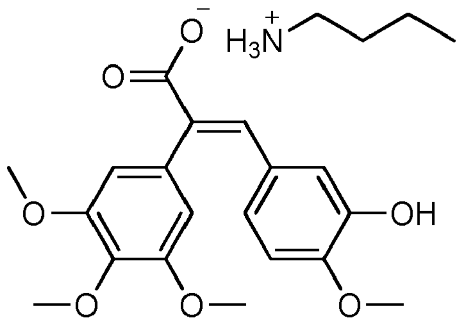

Formula Va.

**16.** A pharmaceutical preparation for treating tumor, **characterized in that** it is a pharmaceutical preparation comprising the compound according to any one of claims 1 to 11, or a salt thereof, or a stereoisomer thereof, or a solvate thereof, as the active ingredient, in combination with pharmaceutically acceptable excipients or auxiliary ingredients; preferably, the pharmaceutical preparation is an injecting preparation, an oral preparation, or an external preparation.

**17.** A pharmaceutical preparation for preventing and/or treating eye disease, **characterized in that** it is a pharmaceutical preparation comprising the compound according to any one of claims 1-5 and 15, or a salt thereof, or a stereoisomer thereof, or a solvate thereof, as the active ingredient, in combination with pharmaceutically acceptable excipients or auxiliary ingredients;

preferably, the pharmaceutical preparation is a preparation administered by ophthalmic delivery;
more preferably, the dosage form of the preparation is eye drops, eye ointment, eye gel, nano preparation, microsphere preparation, and liposome preparation;
further preferably, the eye drops are aqueous solutions, suspensions, and emulsions.

**18.** Compound according to any one of claims 1 to 11, or a salt thereof, or a stereoisomer thereof, or a solvate thereof for use in tumor treatment in combination with tumor immunotherapeutics;
wherein preferably, the tumor immunotherapeutics are PD1, PD-L1, T cells, NK cells, or CART cells.

**19.** The compound for use according to claim 18, **characterized in that** the compound represented by formula Vc, or a salt thereof, or a stereoisomer thereof, or a solvate thereof for use in combination with PD-L1 in the preparation of pharmaceutical compositions for preventing and/or treating lung cancers;

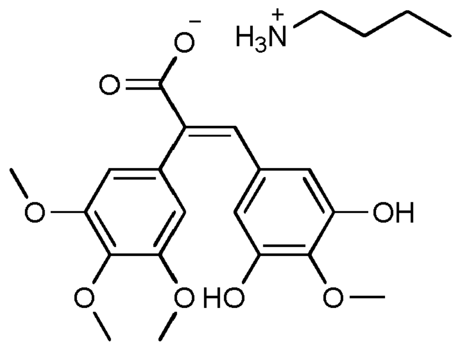

Formula Vc.

**20.** The compound for use according to claim 18, **characterized in that** the compound represented by formula Va, or a salt thereof, or a stereoisomer thereof, or a solvate thereof for use in combination with T cells in the preparation of pharmaceutical compositions for preventing and/or treating liver cancers;

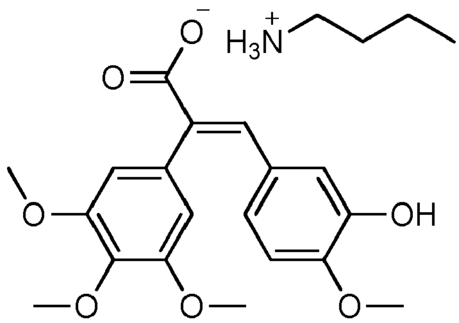

Formula Va.

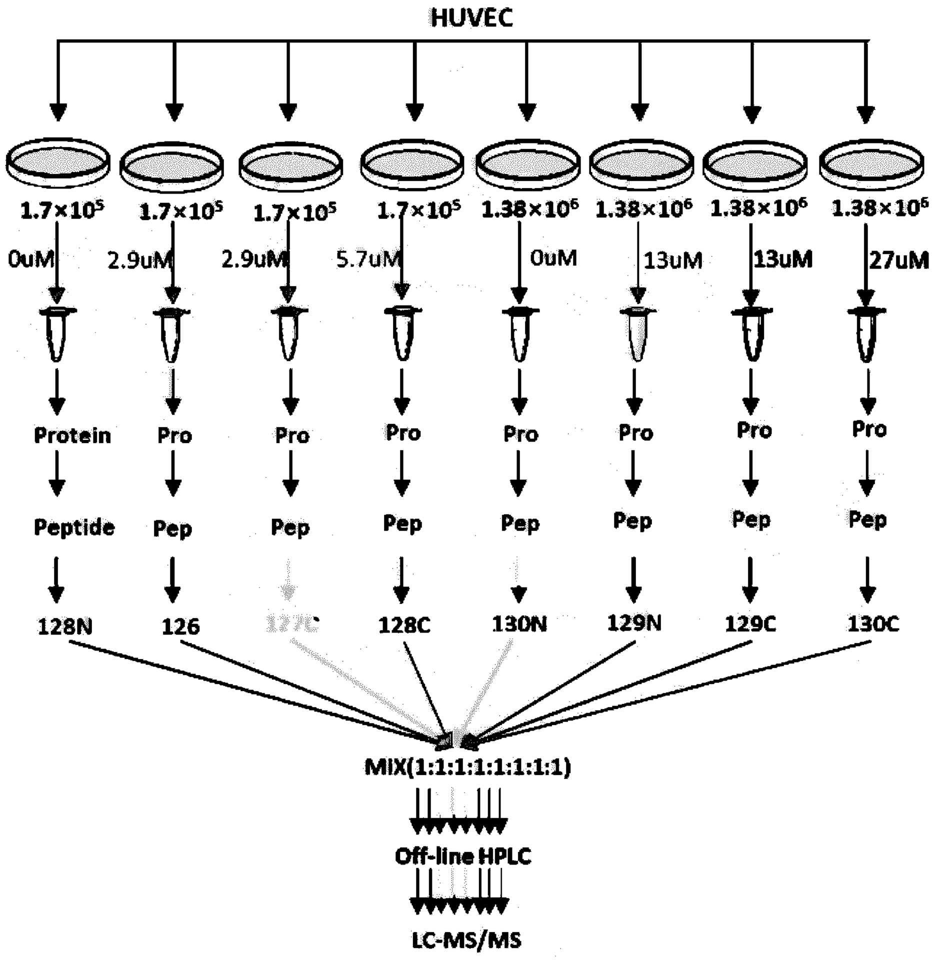
HUVEC
1.7×10^5
1.7×10^5
1.7×10^5
1.7×10^5
1.38×10^6
1.38×10^6
1.38×10^6
1.38×10^6
0uM
2.9uM
2.9uM
5.7uM
0uM
13uM
13uM
27uM
Protein
Pro
Pro
Pro
Pro
Pro
Pro
Pro
Peptide
Pep
Pep
Pep
Pep
Pep
Pep
Pep
128N
126
127C
128C
130N
129N
129C
130C
MIX(1:1:1:1:1:1:1:1)
Off-line HPLC
LC-MS/MS

Figure 1

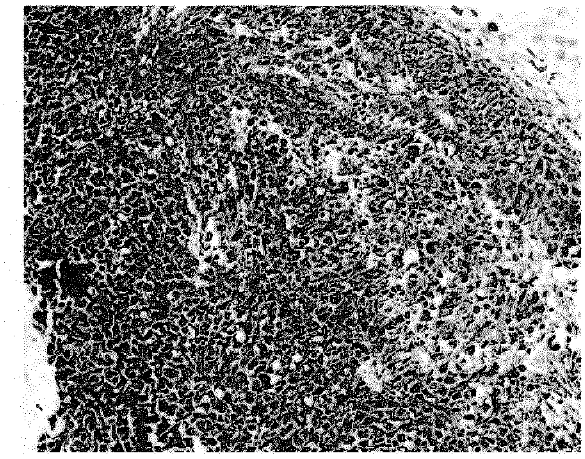

4h Control 100x

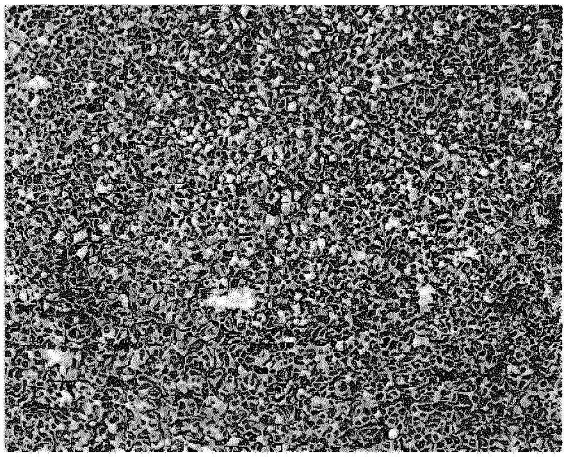

24h Control 100x

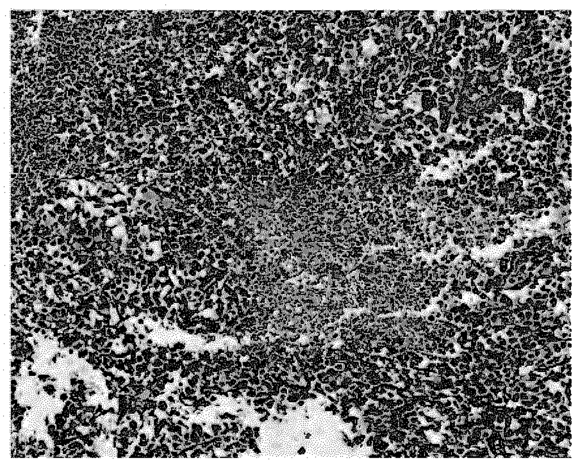

4h Compound **6** 100x

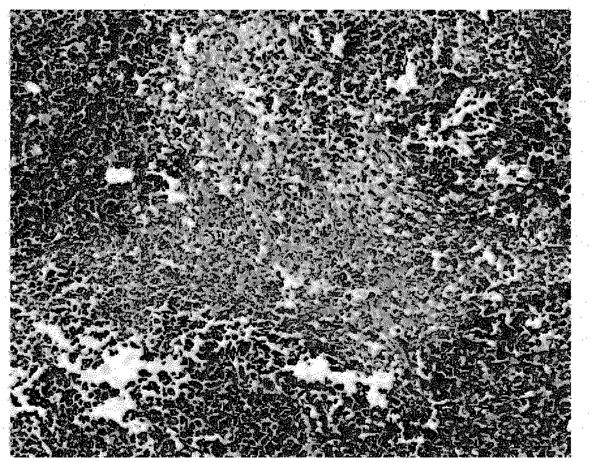

24h Compound **6** 100x

Figure 2

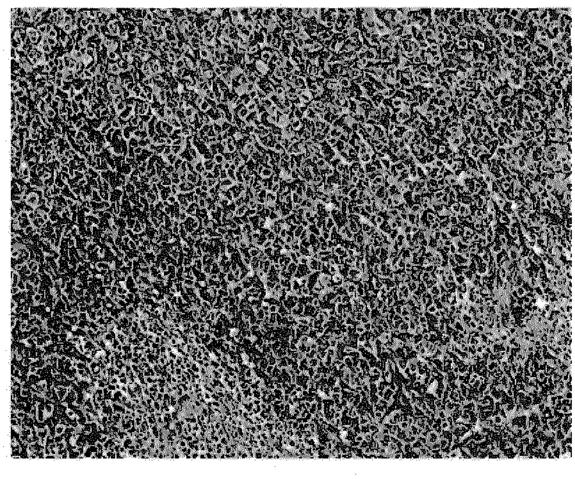

4h Control 100x

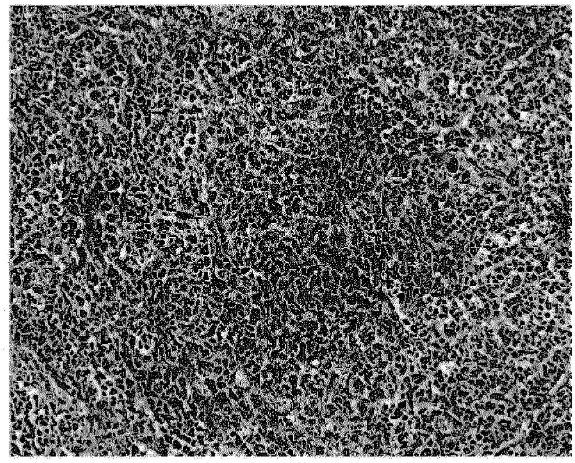

24h Control 100x

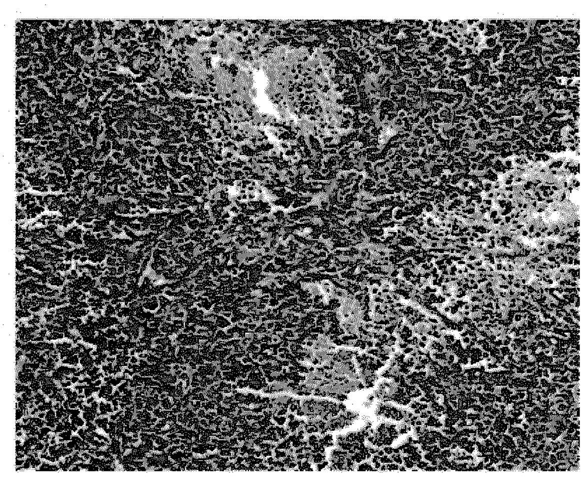

4h Compound **6** 100x

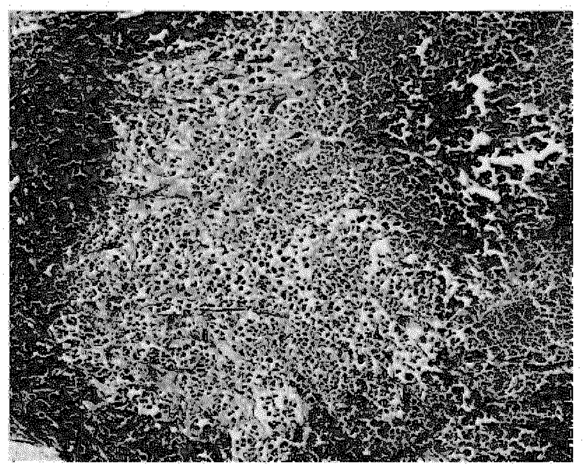

24h Compound **6** 100x

Figure 3

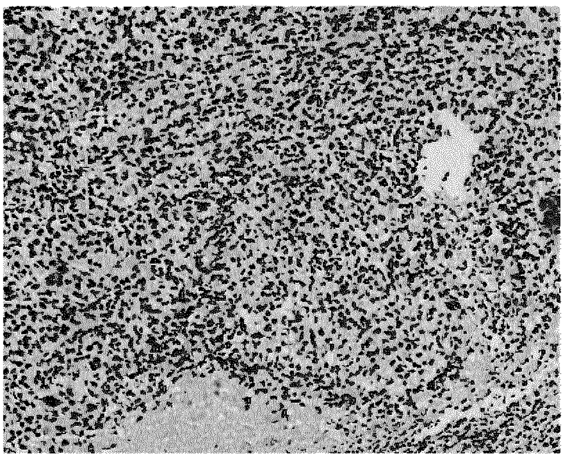

4h Control 100x

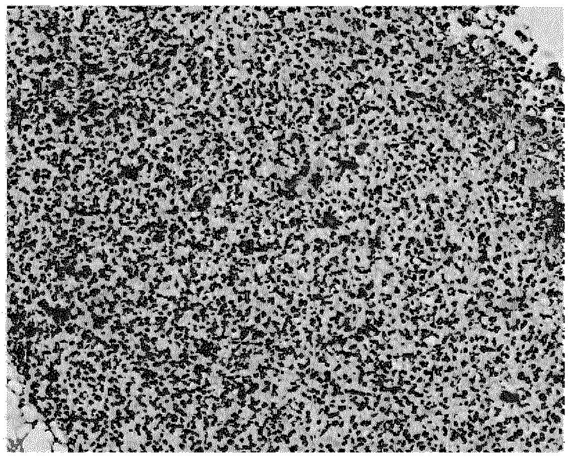

24h Control 100x

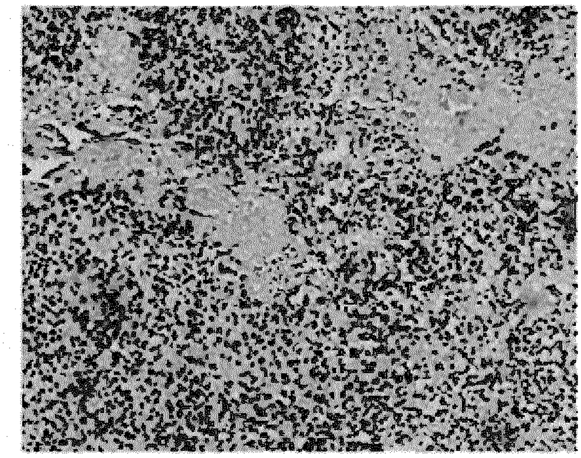

4h Compound 6 100x

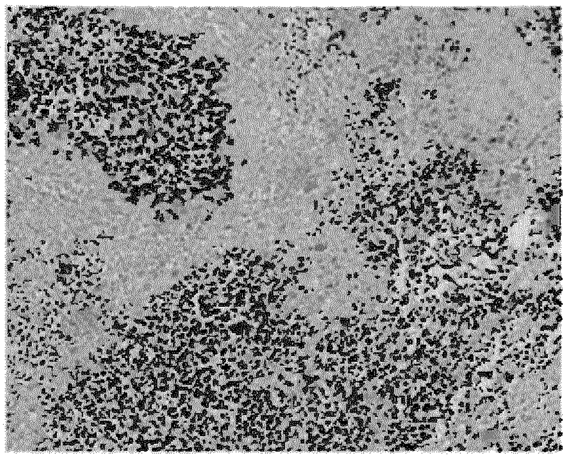

24h Compound 6 100x

Figure 4

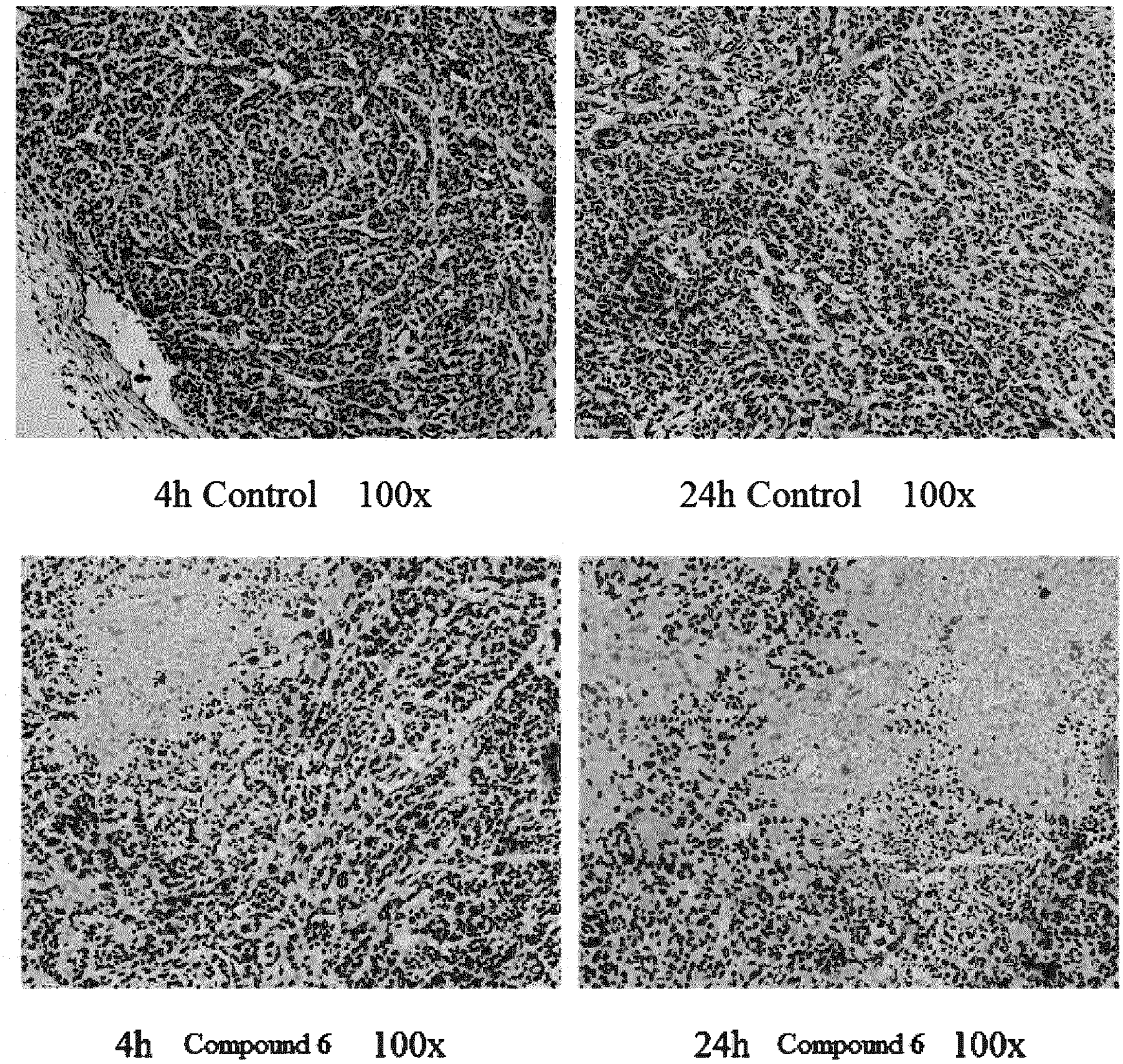


Figure 5

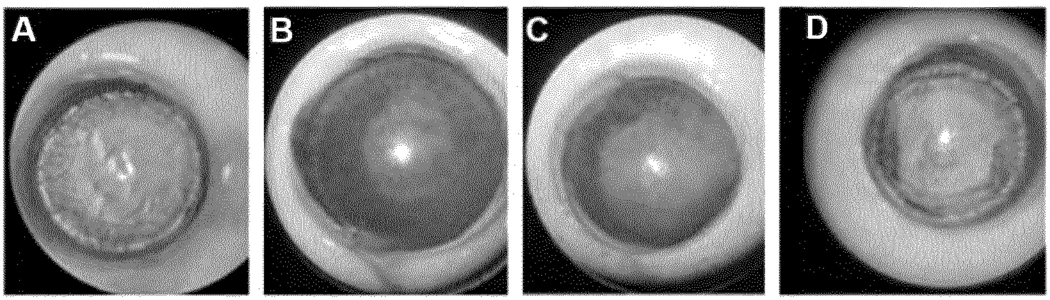


Figure 6

Europäisches Patentamt
European Patent Office
Office européen des brevets

# EUROPEAN SEARCH REPORT

Application Number
EP 23 19 8589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GAZVODA MARTIN ET AL: "2,3-Diarylpropenoic acids as selective non-steroidal inhibitors of type-5 17[beta]-hydroxysteroid dehydrogenase (AKR1C3)", EUROPEAN JOURNAL OF MEDICINAL CHEMISTRY, vol. 62, 1 April 2013 (2013-04-01), pages 89-97, XP093124752, AMSTERDAM, NL ISSN: 0223-5234, DOI: 10.1016/j.ejmech.2012.12.045 | 1-6,9-18 | INV. A61K31/192 A61K9/00 A61P27/02 A61P35/00 A61P35/04 A61K38/17 |
| A | * tables 1,2 * * page 91, left-hand column * ----- | 19,20 | |
| Y | O'BOYLE NIAMH M. ET AL: "Synthesis and evaluation of antiproliferative microtubule-destabilising combretastatin A-4 piperazine conjugates", ORGANIC & BIOMOLECULAR CHEMISTRY, vol. 17, no. 25, 1 January 2019 (2019-01-01), pages 6184-6200, XP093124760, ISSN: 1477-0520, DOI: 10.1039/C9OB00558G | 1-6,9-18 | TECHNICAL FIELDS SEARCHED (IPC) |
| A | * page 6186, scheme 1 * * tables 1,2 * ----- -/-- | 19,20 | A61K A61P |

The present search report has been drawn up for all claims

| Place of search | Date of completion of the search | Examiner |
|---|---|---|
| Munich | 5 February 2024 | Moutafidou, Nikoleta |

1

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

page 1 of 3

Europäisches Patentamt
European Patent Office
Office européen des brevets

# EUROPEAN SEARCH REPORT

Application Number
EP 23 19 8589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MINGYI MA ET AL: "Synthesis and biological evaluation of Combretastatin A-4 derivatives containing a 3'-O-substituted carbonic ether moiety as potential antitumor ag", CHEMISTRY CENTRAL JOURNAL, BIOMED CENTRAL LTD, LO, vol. 7, no. 1, 5 December 2013 (2013-12-05), page 179, XP021171599, ISSN: 1752-153X, DOI: 10.1186/1752-153X-7-179 | 1-6,9-18 | |
| A | * page 3, scheme 1 * <br> * figure 2 * <br> ----- | 19,20 | |
| X | CN 105 566 100 B (DONGGUAN DAXIN BIOLOGICAL TECH CO LTD) 2 November 2018 (2018-11-02) | 1-5, 12-17 | |
| Y | * the whole document * | 1-6,9-18 | |
| A | ----- | 19,20 | TECHNICAL FIELDS SEARCHED (IPC) |
| X | CN 101 074 189 A (YONG ZHIQUAN [CN]) 21 November 2007 (2007-11-21) | 1-6,9, 10,16,17 | |
| Y | * the whole document * | 1-6,9-18 | |
| A | ----- | 19,20 | |
| X | CN 107 773 556 A (DONGGUAN DAXIN BIOLOGICAL TECH CO LTD) 9 March 2018 (2018-03-09) | 7,8 | |
| Y | * claims; examples * | 1-6,9-18 | |
| A | ----- <br> -/-- | 19,20 | |

The present search report has been drawn up for all claims

| Place of search | Date of completion of the search | Examiner |
|---|---|---|
| Munich | 5 February 2024 | Moutafidou, Nikoleta |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 23 19 8589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GREER ALEKSANDRA K. ET AL: "Novel Resveratrol-Based Substrates for Human Hepatic, Renal, and Intestinal UDP-Glucuronosyltransferases", CHEMICAL RESEARCH IN TOXICOLOGY, vol. 27, no. 4, 11 March 2014 (2014-03-11), pages 536-545, XP093099410, US ISSN: 0893-228X, DOI: 10.1021/tx400408x | 1-6,9-18 | |
| A | * the whole document * ----- | 19,20 | |
| | | | TECHNICAL FIELDS SEARCHED (IPC) |

The present search report has been drawn up for all claims

1

| Place of search | Date of completion of the search | Examiner |
|---|---|---|
| Munich | 5 February 2024 | Moutafidou, Nikoleta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 23 19 8589

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2024

| Patent document cited in search report | Publication date | Patent family member(s) | Publication date |
|---|---|---|---|
| CN 105566100 B | 02-11-2018 | NONE | |
| CN 101074189 A | 21-11-2007 | NONE | |
| CN 107773556 A | 09-03-2018 | CN 107773556 A | 09-03-2018 |
| | | EP 3378473 A1 | 26-09-2018 |
| | | JP 6656397 B2 | 04-03-2020 |
| | | JP 2019524637 A | 05-09-2019 |
| | | US 2018353449 A1 | 13-12-2018 |
| | | US 2020246290 A1 | 06-08-2020 |
| | | WO 2018035884 A1 | 01-03-2018 |

EPO FORM P0459

For more details about this annex : see Official Journal of the European Patent Office, No. 12/82